# FASCICULE DE BREVET EUROPEEN

(11) **EP 2 356 257 B1**
(45) Date de publication et mention de la délivrance du brevet: **15.04.2015**
(21) Numéro de dépôt: 09784341.1
(22) Date de dépôt: 17.11.2009
(51) Int. Cl.: C07K 14/005, C07K 14/115, C12Q 1/68

(54) **PROTÉINES MUTANTES DE LA PROTÉINE F DE PIV-5 ET DE PIV-2**
MUTIERTE F PROTEINE VON PIV-5 UND PIV-2
MUTANT F PROTEINS OF PIV-5 ET PIV-2

(30) Priorité: 21.11.2008 FR 0806547
(43) Date de publication de la demande: 17.08.2011
(73) Titulaire: Centre National de la Recherche Scientifique, 75794 Paris Cedex 16 (FR); Université Claude Bernard de Lyon 1, 69622 Villeurbanne Cedex (FR); Les Hospices Civils de Lyon, 69424 Lyon Cedex 03 (FR)
(72) Inventeur: ROSA CALATRAVA, Manuel, Melchior, Jean-Pierre, F-69003 Lyon (FR); TERRIER, Olivier, F-69008 Lyon (FR); DURUPT, François, Edouard, Julien, F-69003 Lyon (FR)
(74) Mandataire: Paris, Fabienne
(86) Numéro de dépôt international: PCT/FR2009/001318
(87) Numéro de publication internationale: WO 2010/058100

(56) Documents cités:
- WO-A-02/077211
- TERRIER O ET AL: "Engineering of a parainfluenza virus type 5 fusion protein (PIV-5 F): Development of an autonomous and hyperfusogenic protein by a combinational mutagenesis approach" VIRUS RESEARCH, vol. 146, no. 1-2, décembre 2009 (2009-12) , pages 115-124, XP002573397 ISSN: 0168-1702
- RUSSELL CHARLES J ET AL: "A dual-functional paramyxovirus F protein regulatory switch segment: Activation and membrane fusion." JOURNAL OF CELL BIOLOGY, vol. 163, no. 2, 27 octobre 2003 (2003-10-27), pages 363-374, XP002533550 ISSN: 0021-9525
- KAWANO M ET AL: "Sequence of the fusion protein gene of human parainfluenza type 2 virus and its 3' intergenic region: Lack of small hydrophobic (SH) gene" VIROLOGY, ACADEMIC PRESS,ORLANDO, US, vol. 178, no. 1, 1 septembre 1990 (1990-09-01), pages 289-292, XP023055964 ISSN: 0042-6822 [extrait le 1990-09-01]

## Description

### DOMAINE TECHNIQUE

La présente demande est relative à des protéines mutantes de la protéine de fusion (protéine F) de virus *Parainfluenza* (PIV) qui sont actuellement répertoriés sous PIV de type 5 (PIV-5 ou PIV5) et PIV de type 2 (PIV-2 ou PIV2).

La présente demande est relative à des produits en dérivant, tels que :
- acides nucléiques, vecteurs, cellules,
- inhibiteurs de fusion de type anticorps, aptamères, ARN interférant,
- myélomes, hybridomes,
- cellules souches et progénitrices.

La présente demande est également relative à ces protéines mutantes et produits en dérivant pour leur utilisation dans des applications médicales et biotechnologiques.

### ARRIÈRE-PLAN TECHNOLOGIQUE

Le virus *Parainfluenza* (PIV) qui est actuellement répertorié sous PIV de type 5 (PIV-5 ou PIV5) est un virus enveloppé du genre *Rubulavirus* de la famille des *Paramyxoviridae.* PIV-5 était précédemment connu sous le nom de SV5 (*Simian Virus* 5), car son isolement a initialement été réalisé à partir de cultures primaires de cellules de singe. L'hôte naturel de PIV-5 semble toutefois être le chien, chez lequel il cause une toux connue sous le nom de toux de chenil.

Par la suite, quelques isolats de PIV-5 ont été obtenus à partir de prélèvements collectés sur des humains, mais qui ont été placés en culture sur des cellules animales. En outre, aucun symptôme ou maladie n'est associé à PIV-5 chez l'être humain. La question de savoir si l'être humain est effectivement un hôte pour PIV-5 est donc à ce jour toujours sujette à débats.

En tout état de cause, le virus PIV-5 est actuellement considéré comme un virus animal.

Le virus *Parainfluenza* (PIV) qui est actuellement répertorié sous PIV de type 2 (PIV-2 ou PIV2) est également un virus enveloppé du genre *Rubulavirus* de la famille des *Paramyxoviridae.*

A ce jour, seuls des isolats humains de PIV-2 ont été identifiés (hPIV-2), de sorte que PIV-2 est considéré comme un virus humain.

Les virus PIV-5 et PIV-2 sont très proches l'un de l'autre, tant en terme de séquences d'acides nucléiques, de séquences protéiques, d'organisation, de structure et de morphologie.

Parmi les virus *Parainfluenza* humains, PIV-2 est le virus le plus proche de PIV-5 qui ait été retrouvé chez l'être humain.

PIV-2 peut être considéré, et est à tout le moins considéré par les inventeurs, comme étant l'équivalent humain de PIV-5.

L'infection par PIV-5 ou PIV-2 conduit à la formation de structures plurinucléaires appelées syncytia, qui résultent de la fusion de cellules de l'hôte infecté.

L'entrée des virus PIV-5 et PIV-2 dans la cellule hôte se réalise par la fusion de l'enveloppe virale avec la membrane cellulaire.

Cette fusion implique deux glycoprotéines virales : la protéine d'attachement hémagglutinine-neuraminidase (HN) et la protéine de fusion (F).

La protéine de fusion F de PIV-5 et PIV-2 est synthétisée sous la forme d'un simple précurseur (F0) et se présente sous la forme d'un homotrimère glycosylé. La protéine de fusion F de PIV-5 et PIV-2 nécessite un clivage protéolytique par des furines de l'hôte pour générer une forme « pré-activée », consistant en deux sous-unités liées par des ponts disulfures : une grande sous-unité F1 carboxy-terminale et une petite sous-unité amino-terminale F2.

La sous-unité F1 est composée d'un peptide de fusion hydrophobe (FP) ainsi que deux domaines répétés en heptades (HR-1 et HR-2), présentant une conformation de type super-enroulé (« *coiled-coil* »). Après activation par HN, la protéine de fusion subit un ensemble de changements conformationnels résultants en l'insertion du peptide de fusion dans la membrane cellulaire cible. S'ensuit l'interaction entre les domaines HR-1 et HR-2 qui rapproche l'enveloppe virale de la membrane cellulaire *(Russell et al.* 2006). Ces domaines son connus pour former un fagot (« *bundle* ») de six hélices très stable constitué d'une structure trimérique super-enroulée (« *coiled-coil* »), dans laquelle trois domaines HR-1 sont liés avec trois domaines HR-2 en orientation antiparallèle. Cette conformation représente la forme de post-fusion de la protéine F (Baker *et al.* 1999, Sergel-Germano *et al.* 2000, West *et al.* 2005).

La protéine de fusion F de PIV-5 et PIV-2 nécessite une protéine HN provenant du même type viral pour qu'il y ait promotion de la fusion (Yao *et al.* 1997). Toutefois, la nature précise des interactions qui existent entre F et HN n'est encore pas bien connue.

Plusieurs études ont néanmoins montré que certaines souches de PIV-5 et de PIV-2 différent dans leur nécessité de HN pour le déclenchement de la fusion.

Par exemple, Ito *et al.* 1997 décrivent deux souches de « SV5 » (PIV-5), à savoir W3A et WR, dont les protéines F ne diffèrent que par trois acides aminés (résidus 22, 443 et 516). La protéine F de la souche W3A est capable d'induire la fusion de manière autonome, c'est-à-dire en l'absence de la protéine HN, alors que la protéine F de la souche WR n'en est pas capable.

Ito *et al.* 1997 indiquent que l'activité fusogène de la protéine F de la souche WR peut être rétablie en remplaçant l'acide aminé en position 22 de cette protéine par un acide aminé Proline.

Ito *et al.* 2000 suggèrent que l'acide aminé E en position 132 et l'acide aminé A en position 290 de la protéine F des souches W3A et WR pourraient être impliqués dans la capacité de la protéine F de ces souches à être autonome de la protéine HN.

Paterson *et al.* 2000 indiquent que la présence de l'acide aminé Proline en position 22 de la protéine F de la souche W3A, et la présence de l'acide aminé 443 de la protéine F de la souche WR pourraient augmenter la capacité fusogène de ces souches virales.

Russell *et al.* 2003 indiquent que le fait remplacer les résidus L447 et I449 de la protéine F de la souche W3A par des acides aminés aromatiques pourrait augmenter l'activité fusogène de la protéine F de cette souche virale.

Gardner et Dutch 2007 indiquent que la mutation I49A de la protéine F d'un virus « SV5 » (PIV-5) de type sauvage aurait un effet pro-fusogène.

Gardner *et al.* 2007 indiquent que la mutation V402A de la protéine F d'un virus « SV5 » (PIV-5) de type sauvage aurait un effet pro-fusogène.

Concernant la protéine PIV-2, il ne semble pas y avoir de document de l'art antérieur qui décrirait l'introduction de mutation(s) dans la séquence de la protéine F de ce virus, de sorte à tenter d'en accroître l'autonomie et la capacité fusogène.

Par ailleurs, il existe de nombreuses autres protéines virales capables de fusion, telles que par exemple les protéines HA1/HA2 de *Influenza,* la protéine G des *Rhabdovirus,* la protéines gp41/gp120 du VIH.

Les connaissances actuelles concernant les capacités de fusogénicité et d'autonomie de ces différentes protéines virales restent à ce jour très limitées.

En tout état de cause, les connaissances actuelles concernant les protéines de fusion virales n'apportent pas une maîtrise technique qui serait suffisante pour en envisager des applications médicales et/ou biotechnologiques effectives.

### RÉSUMÉ DE L'INVENTION

Les inventeurs ont considéré que le fait de disposer d'une protéine de fusion qui serait hyperfusogène et qui ferait également preuve d'une grande autonomie dans sa capacité de fusion permettrait d'apporter une solution à un certain nombre de situations médicales et biotechnologiques.

Les inventeurs ont alors opéré un choix sélectif vis-à-vis de la protéine F de PIV-5 et/ou PIV-2 par rapport à l'ensemble des autres protéines de fusion virales, telles que les protéines HA1/HA2 de *Influenza*, la protéine G des *Rhabdovirus,* la protéines gp41/gp120 du VIH.

Ils ont ensuite construit et produit des protéines F mutantes, qui sont capables de fusogénicité en l'absence de la protéine HN.

Les protéines mutantes de l'invention font preuve d'une forte capacité de fusogénicité et d'une forte autonomie. Elles ne nécessitent pas la présence de la protéine HN pour induire la fusion cellulaire et la formation de syncytia.

Les inventeurs montrent en outre qu'il est possible d'introduire dans ces protéines mutantes un site de clivage différent de celui que présente la protéine F à l'état naturel, et plus particulièrement un site de clivage tissu-spécifique.

Les inventeurs proposent en outre des applications médicales (thérapeutiques, préventives, palliatives, mais aussi diagnostiques) et/ou biotechnologiques des protéines mutantes de l'invention et/ou des produits en découlant ou dérivant.

Plus particulièrement, les inventeurs proposent d'utiliser les protéines mutantes de l'invention ou les acides nucléiques les codant, pour traiter, prévenir ou pallier, par voie *in vivo* ou par voie *ex vivo,* les maladies ou dysfonctions pour lesquelles on souhaite induire ou augmenter la formation de syncytia, telles que les cancers (plus particulièrement les cancers métastatiques, préférentiellement les mélanomes métastatiques) ou les déficiences du développement placentaire.

Les inventeurs proposent en outre des agents bloquant la protéine F de PIV-5 et/ou PIV-2, tels que des anticorps, des cellules dendritiques recombinantes, des antisens, des siRNA, des acides nucléiques aptamères, pour traiter, prévenir ou pallier, par voie *in vivo* ou par voie *ex vivo,* les maladies ou dysfonctions pour lesquelles on souhaite inhiber ou bloquer la formation de syncytia, telles que les infections à virus enveloppé, les allergies, les maladies auto-immunes, les rejets de greffe.

Les inventeurs proposent en outre des moyens diagnostiques pour détecter la formation excessive, ou au contraire insuffisante, de syncytia.

Les inventeurs proposent en outre différents moyens biotechnologiques, notamment pour le criblage de principes actifs capables de diminuer la formation de syncytia.

Les inventeurs proposent en outre des cellules cancéreuses, plus particulièrement des myélomes, qui comprennent une protéine mutante de l'invention. Les inventeurs proposent en outre des hybridomes comprenant une protéine mutante de l'invention.

Les cellules cancéreuses, plus particulièrement les myélomes de l'invention, sont capables de fusionner avec une autre cellule, et plus particulièrement avec un lymphocyte B, sans mettre en oeuvre de polyéthylène glycol (PEG), ni d'électroporation, ni aucun autre moyen inducteur de fusion. Les cellules cancéreuses, plus particulièrement les myélomes de l'invention, sont capables de fusion autonome.

Les hybridomes de l'invention peuvent être produits par une fusion (de lymphocytes B à des myélomes) qui ne requiert pas la mise en oeuvre de polyéthylène glycol (PEG), ni d'électroporation, ni aucun autre moyen inducteur de fusion. En effet, les hybridomes de l'invention peuvent être produits en mettant en oeuvre au moins une cellule cancéreuse, plus particulièrement au moins un myélome de l'invention.

Les inventeurs proposent en outre des cellules souches ou progénitrices recombinantes, qui expriment une protéine mutante de l'invention, et leurs applications dans la production de fibres musculaires.

D'autres aspects de l'invention se trouvent décrits en partie «description détaillée » ci-dessous.

### BRÈVE DESCRIPTION DES FIGURES

Le jeu de figures de la demande telle que déposée a été déposé en couleurs. Il est accessible par consultation du dossier auprès de l'Office.

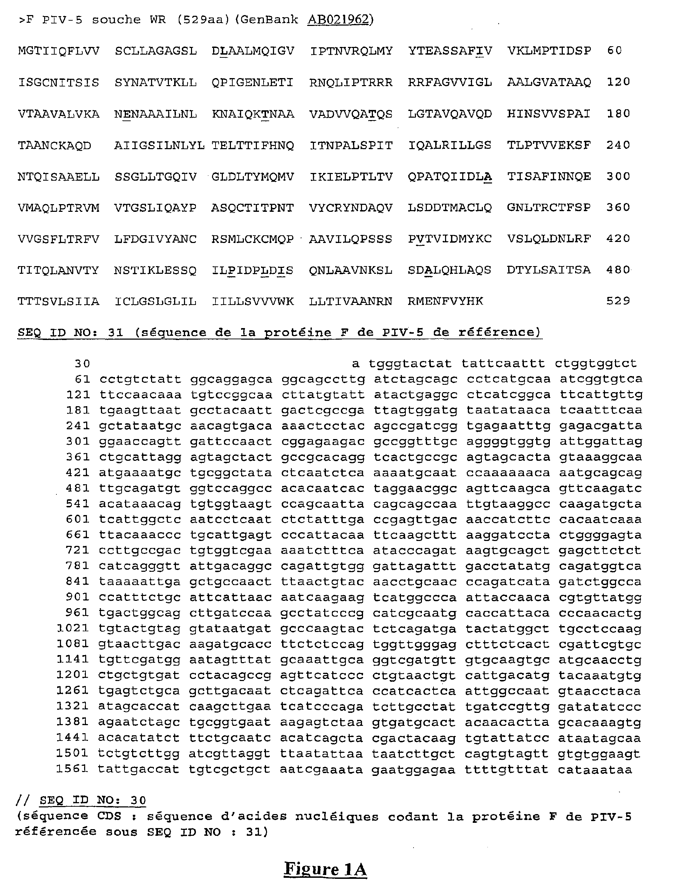

**Figure 1A** **:** séquence de la protéine F de PIV-5 de référence (SEQ ID NO: 31 ; séquence de la protéine F de l'isolat WR) et séquence CDS correspondante (SEQ ID NO : 30 ; séquence d'acides nucléiques codant la protéine de SEQ ID NO : 31).
   En caractères gras et soulignés au sein de la séquence de SEQ ID NO : 31, sont signalés les acides aminés de positions :
   - 22 (L),
   - 49 (I),
   - 132 (E, mutation pré-existante dans cet isolat),
   - 147 (T);
   - 158 (T),
   - 290 (A, mutation pré-existante dans cet isolat),
   - 402 (V),
   - 443 (P, mutation théoriquement pré-existante dans cet isolat),
   - 447 (L),
   - 449 (I), et
   - 463 (A).
**Figure 1B** : séquence de la protéine F de PIV-2 de référence (SEQ ID NO: 33 ; séquence de la protéine F de l'isolat Greer) et séquence CDS correspondante (SEQ ID NO : 32 ; séquence d'acides nucléiques codant la protéine de SEQ ID NO : 33).
   En caractères gras et soulignés au sein de la séquence de SEQ ID NO : 33, sont signalés les acides aminés de positions :
   - 24 (I),
   - 53 (I),
   - 133 (K),
   - 151 (T),
   - 162 (S),
   - 294 (I),
   - 406 (A),
   - 428 (S),
   - 439 (S),
   - 445 (I),
   - 474 (S).
**Figure 2A** : alignement de la protéine F de PIV-5 (acides aminés 4 à 529 de SEQ ID NO: 31) sur celle de PIV-2 (acides aminés 8 à 533 de SEQ ID NO: 33) : l'identité protéique est de 47,7% entre ces deux protéines. La séquence consensus résultant de cet alignement est référencée sous SEQ ID NO: 34.
**Figure 2B** **:** acides aminé(s) et mutation(s) au sein de la protéine F de PIV-2 qui correspondent à ceux de la protéine F de PIV-5

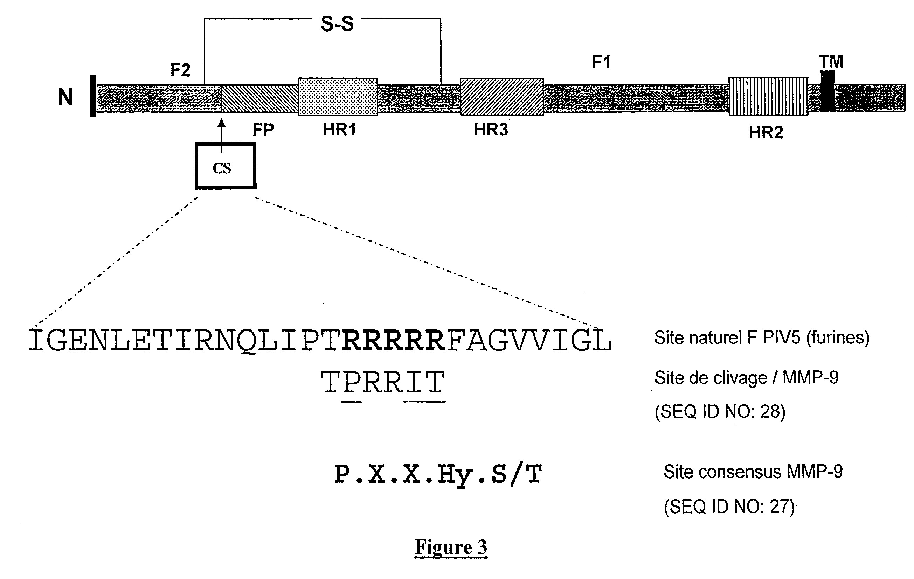

**Figure 3** **:** illustration de la substitution du site de clivage naturel (SEQ ID NO: 23) de la protéine F de PIV-5 par un site de clivage tissu-spécifique, en l'occurrence par le site d'une enzyme exprimée spécifiquement par le tissu tumoral métastatique, à savoir la métallo-protéase matricielle 9 (MMP-9).
   Site consensus d'un site MMP-9 : séquence de SEQ ID NO: 27 (site PXXhyS/T avec X = n'importe quel acide aminé et Hy = n'importe quel acide aminé hydrophobe, c'est-à-dire n'importe quel acide aminé parmi F, M, V, L, I).
   Séquence illustrative d'un site MMP-9 : séquence de SEQ ID NO: 28, séquence de SEQ ID NO : 29.

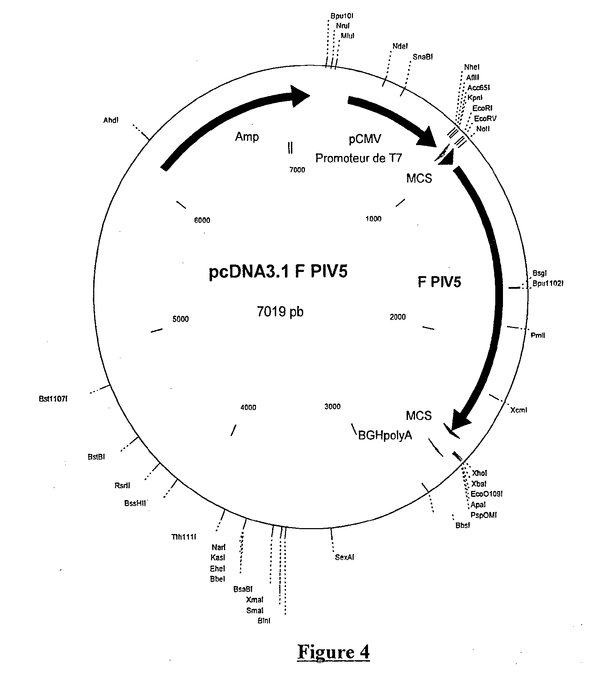

**Figure 4** **:** structure du plasmide pcDNA3.1 sur lequel a été clonée la séquence codant la protéine F de PIV-5.
   Amp : gène de résistance à l'ampicilline
   pCMV : promoteur du Cytomégalovirus
   MCS : site de clonage multiple (*Multiple Cloning Site*)
   F PIV5 : protéine F de PIV-5
   BGHpolyA : signal de poly-adénylation de l'hormone de croissance bovine (*Bovine Growth Hormone polyA*)

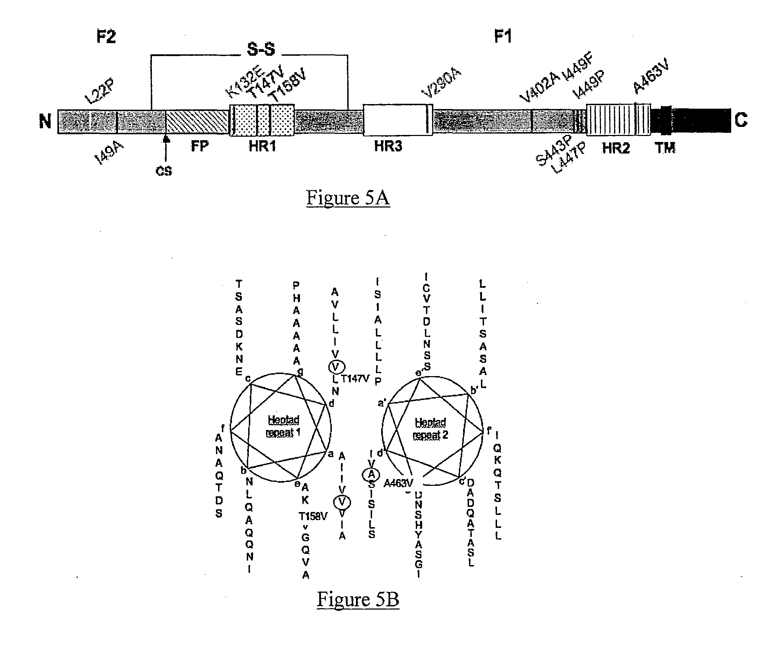

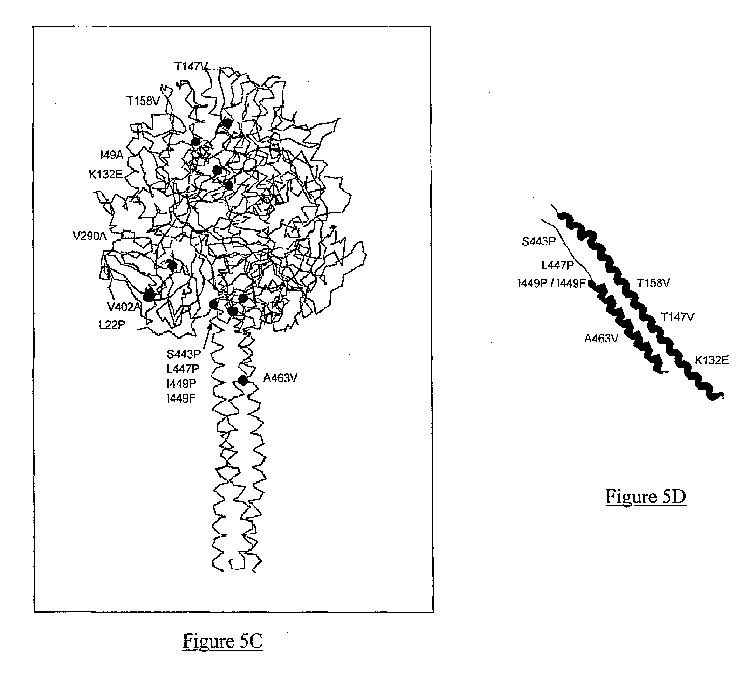

**Figures 5A, 5B****,** **5C, 5D** : visualisation des mutations réalisées par les inventeurs dans la protéine F de PIV-5

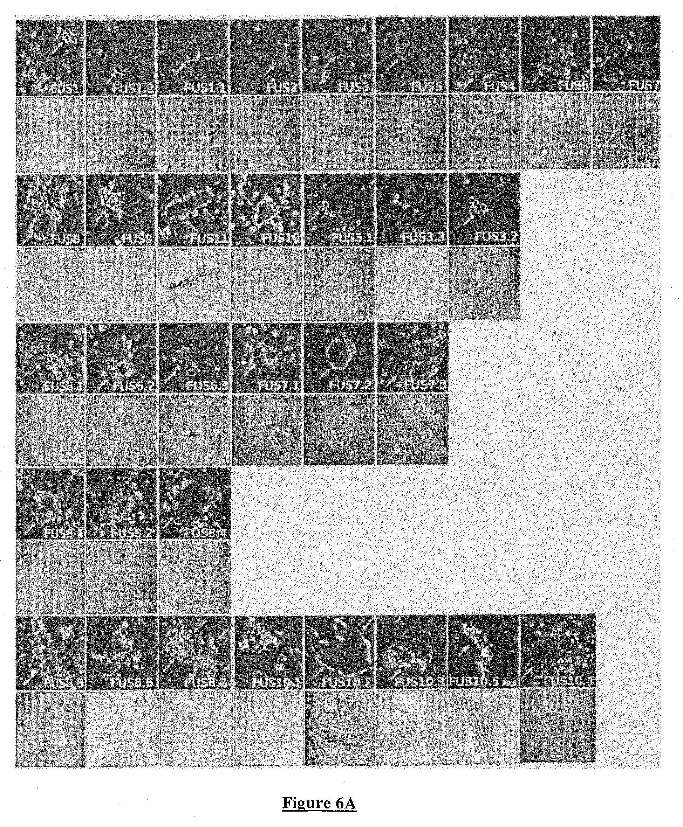

**Figure 6A** : illustration des observations faites au microscope lors des tests de fusion semi-quantitatifs (panel large de mutants réalisés par les inventeurs)

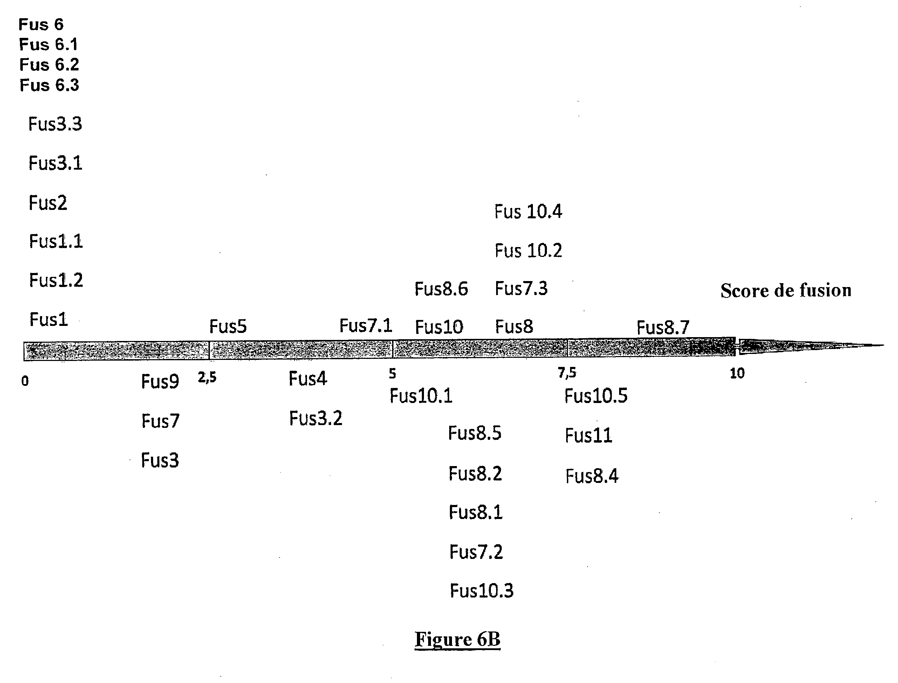

**Figure 6B** **:** diagramme présentant les scores de fusion obtenus à l'issue des tests de fusion semi-quantitatifs (panel large de mutants réalisés par les inventeurs)

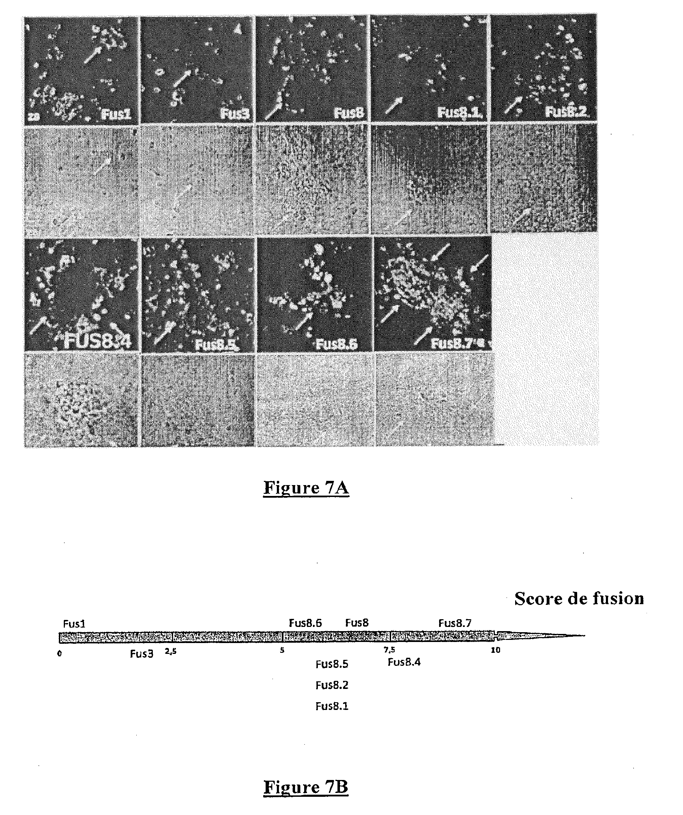

**Figure 7A** **:** illustration des observations faites au microscope lors des tests de fusion semi-quantitatifs (sélection de mutants réalisés par les inventeurs)
**Figure 7B** **:** diagramme présentant les scores de fusion obtenus à l'issue des tests de fusion semi-quantitatifs (sélection de mutants réalisés par les inventeurs)

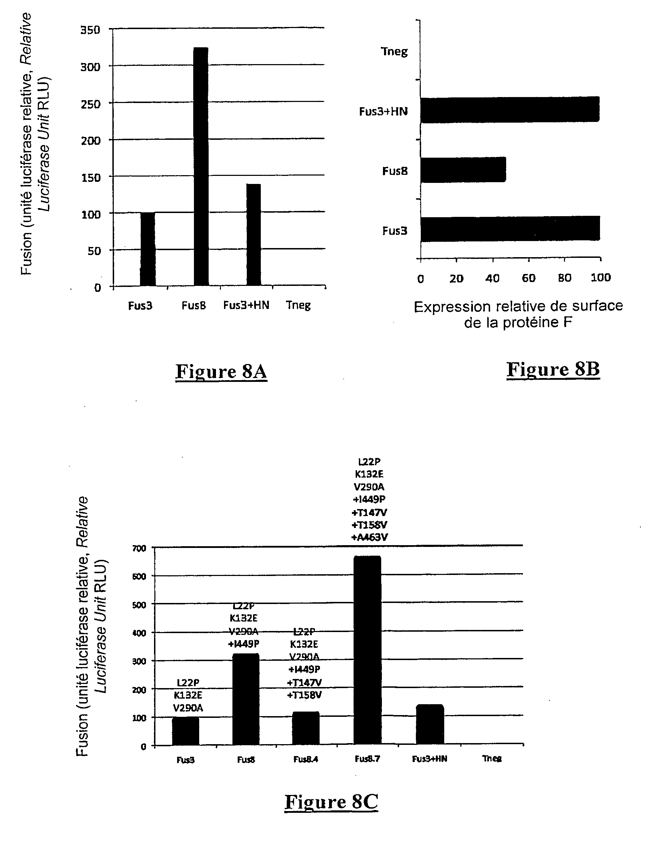

**Figures 8A, 8B**, **8C** : résultats des tests de fusion quantitatifs (sélection de mutants réalisés par les inventeurs)

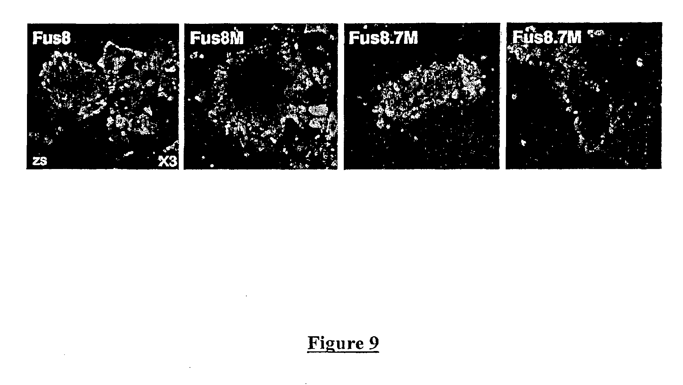

**Figure 9** **:** illustrations des observations faites sous immunofluorescence en microscopie confocale pour des protéines mutantes de l'invention présentant un site de clivage autre que le site de clivage naturel de la protéine F de PIV5 (protéines mutantes Fus8M et Fus8.7M de l'invention, qui dérivent des protéines mutantes Fus8 et Fus8.7 de l'invention par remplacement du site de clivage naturel par le site de clivage MMP-9 de SEQ ID NO: 28).

### DESCRIPTION DÉTAILLÉE

Dans la présente demande, le terme protéine englobe dans sa portée le terme glycoprotéine. C'est notamment le cas pour les protéines F et HN qui sont en fait des glycoprotéines.

### Protéine F de PIV-5 et PIV-2 (protéine non mutante) :

Une séquence de la protéine F de PIV-5 est présentée en Figure 1A (séquence protéique de SEQ ID NO: 31 ; séquence acide nucléique codante de SEQ ID NO: 30). Il s'agit de la séquence de l'isolat WR, qui est un isolat simien. Ces séquences sont celles disponibles dans la base de données Genbank sous le numéro AB021962.

L'échantillon d'isolat WR que les inventeurs ont reçu de l'ATCC et qu'ils ont utilisé pour la construction et la production des protéines mutantes décrites dans les exemples ci-dessous ne présentait toutefois pas l'acide aminé P en position 443 de la protéine F (contrairement à ce qui était attendu au vu de la séquence disponible dans Genbank), mais l'acide aminé S. Cette séquence alternative de la protéine F de l'isolat WR est donc identique à la séquence de SEQ ID NO: 31, à l'exception de l'acide aminé en position 443 qui est S et non P. Par souci de concision, cette séquence alternative sera ici notée « SEQ ID NO: 31 avec S en 443 ».

La séquence de SEQ ID NO: 31 et la séquence alternative « SEQ ID NO : 31 avec S en 443 », préférentiellement la séquence alternative «SEQ ID NO: 31 avec S en 443 », servent de référence de séquence(s) de protéine F de PIV-5 dans le cadre de la présente demande de brevet.

Ceci étant, il existe bien sûr des isolats de PIV-5 autres que l'isolat WR, et notamment :
- d'autres isolats simiens, tels que par exemple l'isolat W3A,
- des isolats d'autres animaux non-humains, tels que :
   o des isolats canins, par exemple les isolats canins CPI+, CPI-, H221, 78524, T1,
   o des isolats porcins, par exemple l'isolat porcin SER,
- des isolats dits « humains » qui sont issus de prélèvements effectués sur des êtres humains mais qui ont été placés en culture sur des cellules animales (*cf.* partie introductive ci-dessus), tels que l'isolat MIL, l'isolat DEN, l'isolat LN, l'isolat MEL, et l'isolat qui, dans WO 02 077211, est décrit comme étant un « cryptovirus ».

Les variations de séquences des protéines F de ces différents isolats PIV-5 sont très faibles.

Une description de ces variations est donnée dans Chatziandreou *et al.* 2004, dont le contenu, et plus particulièrement le tableau 3 et les commentaires qui y sont associés au cours de cet article, sont incorporés par référence dans la présente demande de brevet.

Le tableau 3 de cet article est ici reproduit :

**Tableau 1 (reproduit de l'article Chatziandreou et al. 2004) :**

| **Souche** | | | | | | | | | | | | | **aa** |
|---|---|---|---|---|---|---|---|---|---|---|---|---|---|
| **W3A** (SEQ IDNO: 35) | **WR** (SEQ ID NO : 31) | **MIL** (SEQ IDNO: 36) | **DEN** (SEQ ID NO : 37) | **LN** (SEQ ID NO : 38) | **MEL** (SEQ IDNO: 39) | **Cryptovirus** (SEQ ID NO : 40) | **CPI+** (SEQ IDNO: 41) | **CPI-**(SEQ ID NO : 42) | **H221** (SEQ ID NO : 43) | **78524** (SEQ ID NO : 44) | **T1** (SEQ ID NO : 45) | **SER** (SEQ ID NO : 46) | |
| G | | | | | | S | | | | | | | 2 |
| T | | I | I | I | I | | | | | | | | 3 |
| I | | | | | | | | | R | R | R | | 4 |
| F | | | | | | S | | | | | | | 7 |
| A | | | | | | | | | S | S | T | | 17 |
| S | | G | G | G | G | | | | | | | | 19 |
| P | L | | | | | | | | | | | | 22 |
| S | | | | | | | P | P | | | | | 71 |
| V | | | | | | | | | | | M | | 76 |
| N | | | | | | | | | Y | Y | Y | | 92 |
| E | | | | | | | K | K | K | K | K | K | 132 |
| A | | | | | | T | | | | | | | 134 |
| A | | | | | | | | | V | V | V | | 135 |
| A | | | | | | | | | | | | T | 149 |
| V | | | | | | I | | | | | | | 176 |
| I | | | | M | | | | | | | | | 271 |
| T | | | | | | | | A | | | | | 279 |
| A | | | | | | | | | | | V | | 290 |
| T | | | | | | | K | K | | | | | 307 |
| M | | | | | | I | I | I | I | I | I | I | 310 |
| M | | | | | R | | | | | | | | 346 |
| L | | | | F | | | | | | | | | 366 |
| V | | | | | | | | | | | | M | 370 |
| Y | | | | | | | | | F | F | | | 377 |
| M | | | | | | | I | I | | | | | 407 |
| Y | | | | | | H | H | H | | | | | 408 |
| N | | | | | | D | | | | | | | 417 |
| F | | | | | | | L | L | | | | | 420 |
| V | | | | | | | | | | | I | | 428 |
| S | | | | | | T | T | T | T | T | T | T | 438 |
| S | P | P | P | P | P | P | P | P | P | P | P | P | 443 |
| H | | | | | | | N | N | | | | | 451 |
| I | | | | | | | | | | | | M | 489 |
| L | | F | F | F | F | | | | | | | | 498 |
| L | | | | | | | S | S | | | | | 500 |
| V | | | | | | | A | A | | | | | 507 |
| K | | | | | | | R | R | | | | | 510 |
| V | A | A | A | A | A | A | A | A | A | A | A | T | 516 |
| K | | N | N | N | N | N | N | N | N | N | N | N | 529 |
| Stop | Stop | Q | | | | S | S | S | S | S | S | S | 530 |
| | | H | | | | | Y | Y | | | | | 533 |
| | | S | | | Stop | P | | | | | | | 535 |
| | | Q | | | | R | R | R | R | R | R | R | 536 |

| | | | | | | | | | | | | | |
|---|---|---|---|---|---|---|---|---|---|---|---|---|---|
| aa : position de l'acide aminé | | | | | | | | | | | | | |

Dans le tableau 1 ci-dessus, une case vide indique que la protéine F concernée présente le même acide aminé que la protéine F de la souche W3A indiquée en colonne de gauche.

Les acides aminés dont les positions ne sont pas expressément listées dans ce tableau sont bien sûr identiques à ceux qui leur correspondent dans la séquence de la protéine F de W3A. Ces acides aminés sont eux-mêmes identiques à ceux qui leur correspondent dans la séquence de la protéine F de l'isolat WR (*cf.* séquence de SEQ ID NO: 31).

Ainsi, les séquences de SEQ ID NO: 35 à 46 sont les séquences qui résultent du remplacement dans la séquence de SEQ ID NO: 31 des acides aminés indiqués dans le tableau 1 pour chacune de ces séquences (et, le cas échéant, de l'ajout à la séquence de SEQ ID NO: 31 des acides aminés indiqués en partie C-terminale).

La protéine F de l'isolat W3A, ainsi que celle des autres isolats ci-dessus mentionnés, présente :
- en position 147, l'acide aminé T,
- en position 158, l'acide aminé T,
- en position 447, l'acide aminé L, et
- en position 449, l'acide aminé I.

On voit ainsi que les isolats W3A et WR ne présentent pas l'extension cytoplasmique qui, dans les autres isolats, s'étend au-delà de la position 529. Selon l'isolat concerné, cette extension cytoplasmique contient de deux à sept acides aminés.

On voit également que les séquences des protéines F de ces isolats varient de moins de 5% (plus particulièrement, d'au maximum 3%) par rapport à la séquence de la protéine F de la souche WR (sans tenir compte de l'extension cytoplasmique, c'est-à-dire en calculant ce pourcentage sur la longueur de la protéine F de WR) ; *cf.* fin de page 85 de l'article Chatziandreou *et al.* 2004.

Une protéine F de PIV-5 peut donc consister en :
- la séquence de SEQ ID NO: 31, ou ladite séquence alternative « SEQ ID NO: 31 avec S en 443 », ou en
- une séquence variante de cette séquence de SEQ ID NO: 31 ou de cette séquence alternative « SEQ ID NO: 31 avec S en 443 », cette séquence variante pouvant être définie comme :
   o étant d'une taille identique à celle de SEQ ID NO: 31 ou inférieure d'un maximum de 7 acides aminés à celle de SEQ ID NO: 31 ou supérieure d'un maximum de 7 acides aminés à celle de SEQ ID NO: 31 [ladite séquence alternative « SEQ ID NO: 31 avec S en 443 » présente la même taille que la séquence de SEQ ID NO: 31], préférentiellement d'une taille identique à celle de SEQ ID NO: 31 ou supérieure d'un maximum de 7 acides aminés à celle de SEQ ID NO: 31, et
   o présentant une identité de séquence de plus de 95%, préférentiellement d'au moins 96%, plus préférentiellement d'au moins 97%, par rapport à la séquence de SEQ ID NO: 31 ou à ladite séquence alternative «SEQ ID NO: 31 avec S en 443 » (cette identité étant calculée sur la longueur de la séquence de SEQ ID NO: 31 ou, le cas échéant, de ladite séquence alternative « SEQ ID NO: 31 avec S en 443 »).

Les séquences variantes de la protéine F de PIV-5 de SEQ ID NO: 31 comprennent notamment les séquences des protéines F des isolats W3A, MIL, DEN, LN, MEL, cryptovirus, CPI+, CPI-, H221, 78524, T1 et SER mentionnés ci-dessus (*cf.* tableau 1 ci-dessus et article Chatziandreou *et al.* 2004).

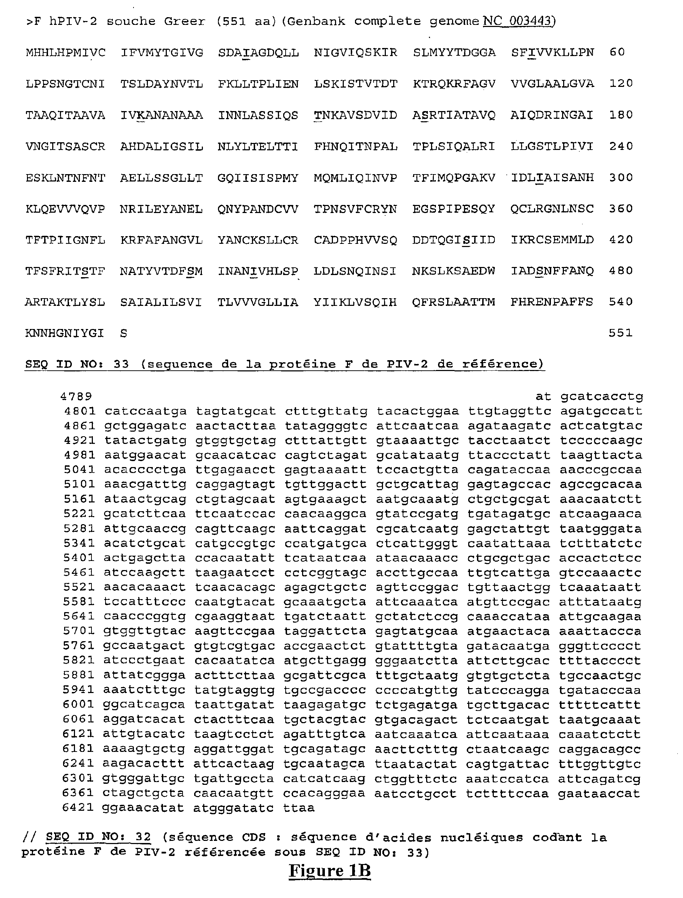

De manière similaire, la séquence qui dans la présente demande sert de référence pour la protéine F de PIV-2 est la séquence de la souche Greer qui est présentée en Figure 1B (séquence protéique de SEQ ID NO: 33, séquence acide nucléique codante de SEQ ID NO: 32).

Il existe bien sûr des isolats PIV-2 autres que l'isolat Greer, tels que par exemple les isolats Toshiba, V98, V94.

La séquence de la protéine F de ces autres isolats PIV-2 est très proche de celle de l'isolat Greer, mais présente quelques petites variations qui sont des variations inter-isolats. Ainsi, une protéine F de PIV-2 peut donc consister en :
- la séquence de SEQ ID NO: 33, ou en
- une séquence variante de cette séquence de SEQ ID NO: 33, cette séquence variante pouvant être définie comme :
   o étant d'une taille identique à celle de SEQ ID NO: 33 ou inférieure d'un maximum de deux acides aminés à celle de SEQ ID NO: 33 ou supérieure d'un maximum de deux acides aminés à celle de SEQ ID NO: 33, préférentiellement d'une taille identique à celle de SEQ ID NO: 33, préférentiellement étant d'une taille identique à celle de SEQ ID NO: 33, et
   o présentant une identité de séquence de plus de 95%, préférentiellement d'au moins 96%, plus préférentiellement d'au moins 97%, par rapport à la séquence de SEQ ID NO: 33 (cette identité étant calculée sur la longueur de la séquence de SEQ ID NO: 33).

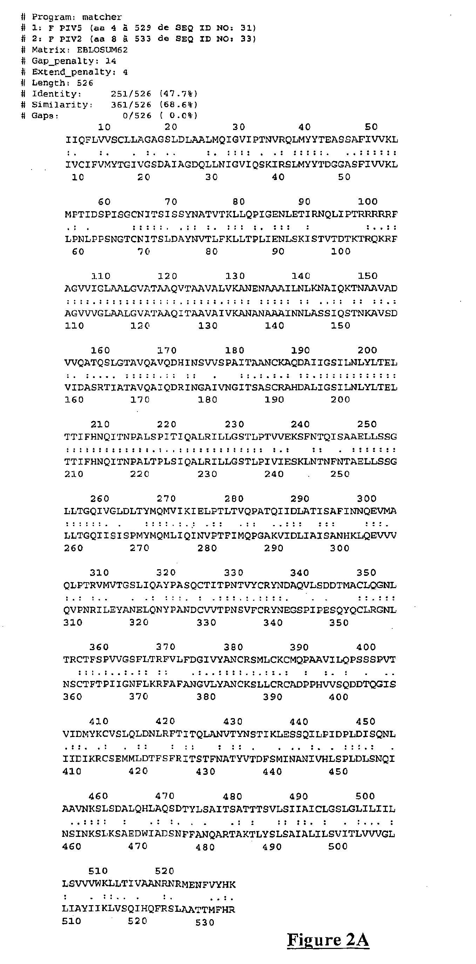

La séquence consensus (SEQ ID NO: 34) résultant de l'alignement de la séquence de la protéine F de PIV-5 (SEQ ID NO: 31) sur celle de PIV-2 (SEQ ID NO: 33) est la suivante peut être lue en figure 2A. Cette séquence consensus peut être re-transcrite comme suit :

I----V------G--------L--IGVI----R-LMYYT-----FIVVKL
-P--------CNITS---YN-T--KLL-P—-ENL--I---------R-RF
AGVV-GLAALGVATAAQ-TAAVA-VKAN-NAAAI-NL---IQ-TN-AV-D
V-A------TAVQA-QD-IN------IT-A-C-A-DA-IGSILNLYLTEL
TTIFHNQITNPAL-P--IQALRILLGSTLP-V-E---NT----AELLSSG
LLTGQI------YMQM-I-I-PT----QP----IDL—-ISA----QEV--
Q-P-R--------Q-YPA—C--TPN-V-CRYN----------CL-GNL
--CTF-P--G-FL-RF----G--YANC-S-LC-C—P—V--Q------
-ID---C----LD---F-IT---N-TY----------I---PLD-S---
---NKSL--A----A-S---------A-T---LS-IA-L-----L----L
L-----KL-------R--------H- (SEQ ID NO: 34),

le symbole "-" indiquant que les protéines F de PIV-5 et de PIV-2 ont à cette position des acides amines différents.

Cette séquence consensus peut également être formalisée comme suit : 
IXXXXVXXXXXXGXXXXXXXXLXXIGVIXXXXRXLMYYTXXXXXFIVVKL
XPXXXXXXXXCNITSXXXYNXTXXKLLXPXXENLXXIXXXXXXXXXXRXRF
AGVVXGLAALGVATAAQXTAAVAXVKANXNAAAIXNLXXXIQXTNXAVXD
VXAXXXXXXTAVQAXQDXINXXXXXXITXAXCXAXDAXIGSILNLYLTEL
 
TTIFHNQITNPALXPVXIQALRILLGSTLPXVXEXXXNTXXXXAELLSSG
LLTGQIXXXXXXYMQMXIXIXPTXXXXQPXXXXIDLXXISAXXXXQEVXX
QXPXRXXXXXXXXQXYPAXXCXXTPNXVXCRYNXXXXXXXXXXXCLXGNL
XXCTFXPXXGXFLXRFXXXXGXXYANCXSXLCXCXXPXXVXXQXXXXXXX
XIDXXXCXXXXLDXXXFXITXXXNXTYXXXXXXXXXXXIXXXXPLDXSXXX
XXXNKSLXXAXXXXAXSXXXXXXXXXXAXTXXXLSXIAXLXXXXXLXXXXL
LXXXXXKLXXXXXXXRXXXXXXXXXHX (SEQ ID NO: 34),
 avec X = n'importe quel acide aminé.

La séquence de la protéine F de l'isolat WR de PIV-5 est la séquence de SEQ ID NO: 34 précédée des acides aminés MGT à l'extrémité N-terminale (*cf.* Figures 1A et 2A).

La séquence de la protéine F de l'isolat Greer de PIV-2 est la séquence de SEQ ID NO: 34, précédée des acides aminés MHHLHPM (SEQ ID NO: 86) à l'extrémité N-terminale et suivie des acides aminés ENPAFFSKNNHGNIYGIS (SEQ ID NO: 87) à l'extrémité C-terminale (*cf.* Figures 1B et 2A).

La séquence des protéines F de PIV-5 et PIV-2 peut être considérée comme étant une séquence comprenant la séquence de SEQ ID NO: 34, préférentiellement comme étant la séquence d'une protéine F de virus PIV qui comprend la séquence de SEQ ID NO: 34. Plus particulièrement, la séquence des protéines F de PIV-5 et PIV-2 peut être considérée comme étant :
a) la séquence de SEQ ID NO: 34 :
   ▪ précédée de 3 à 7 acides aminés à l'extrémité N-terminale, plus particulièrement de 3 acides aminés (tels que MGT) ou de 7 acides aminés (tels que MHHLHPM) à l'extrémité N-terminale, et
   ▪ éventuellement suivie de 18 acides aminés à l'extrémité C-terminale, plus particulièrement des acides aminés ENPAFFSKNNHGNIYGIS à l'extrémité C-terminale, ou
b) une séquence variante de la séquence décrite sous a) ci-dessus, ladite séquence variante étant :
   ▪ soit :
      i. d'une taille identique à celle de SEQ ID NO: 31 ou inférieure d'un maximum de 7 acides aminés à celle de SEQ ID NO: 31 ou supérieure d'un maximum de 7 acides aminés à celle de SEQ ID NO: 31, préférentiellement d'une taille identique à celle de SEQ ID NO: 31 ou supérieure d'un maximum de 7 acides aminés à celle de SEQ ID NO: 31, et
      ii. présentant une identité de séquence de plus de 95%, préférentiellement d'au moins 96%, plus préférentiellement d'au moins 97%, par rapport à la séquence de SEQ ID NO: 31 ou à ladite séquence alternative « SEQ ID NO: 31 avec S en 443 » (cette identité étant calculée sur la longueur de la séquence de SEQ ID NO: 31 ou, le cas échéant, de ladite séquence alternative « SEQ ID NO: 31 avec S en 443 ») ;
   ▪ soit :
      i. d'une taille identique à celle de SEQ ID NO: 33 ou inférieure d'un maximum de deux acides aminés à celle de SEQ ID NO: 33 ou supérieure d'un maximum de deux acides aminés à celle de SEQ ID NO: 33, préférentiellement d'une taille identique à celle de SEQ ID NO: 33, et
      ii. présentant une identité de séquence de plus de 95%, préférentiellement d'au moins 96%, plus préférentiellement d'au moins 97%, par rapport à la séquence de SEQ ID NO: 33 (cette identité étant calculée sur la longueur de la séquence de SEQ ID NO: 33).

### Protéines mutantes de l'invention :

La présente demande est relative à une protéine mutante, dont la séquence en acides aminés comprend une séquence qui est susceptible de dériver de celle de la protéine F d'un virus PIV-5 ou PIV-2 :
- par remplacement :
   - de l'acide aminé qui, dans la séquence de ladite protéine F de virus PIV-5, est en position 22, ou qui, dans la séquence de ladite protéine F de virus PIV-2, est en position 24, par l'acide aminé P (mutation 22P dans F de PIV-5 ; mutation 24P dans F de PIV-2), et
   - de l'acide aminé qui, dans la séquence de ladite protéine F de virus PIV-5, est en position 132, ou qui, dans la séquence de ladite protéine F de virus PIV-2, est en position 133 (mutation 132E dans F de PIV-5 ; mutation 133E dans F de PIV-2), par l'acide aminé **E,** et
   - de l'acide aminé qui, dans la séquence de ladite protéine F de virus PIV-5, est en position **290,** ou qui, dans la séquence de ladite protéine F de virus PIV-2, est en position 294, par l'acide aminé A (mutation 290A dans F de PIV-5 ; mutation 294A dans F de PIV-2), et
   - de l'acide aminé qui, dans la séquence de ladite protéine F de virus PIV-5, est en position **449,** ou qui, dans la séquence de ladite protéine F de virus PIV-2, est en position 439, par l'acide aminé **P** (mutation 449P dans F de PIV-5 ; mutation 439P dans F de PIV-2),
- et, optionnellement :
   - par substitution du site de clivage natif (ou naturel) de ladite protéine F par un autre site de clivage enzymatique, et/ou par insertion dans ladite protéine F d'un site de clivage enzymatique autre que le site de clivage natif (ou naturel) de cette protéine F, et/ou
   - par délétion d'une partie C-terminale de ladite protéine F, ladite partie C-terminale s'étendant en direction N-terminale à partir du dernier acide aminé à l'extrémité C-terminale de la protéine, mais sans s'étendre au-delà du domaine HR2 de ladite protéine F.

Lesdites positions d'acides aminés sont calculées par rapport à la séquence de la forme précurseur (F0) de ladite protéine F (c'est-à-dire la séquence de la protéine F avant clivage), en comptant les positions de l'extrémité N-terminale vers l'extrémité C-terminale.

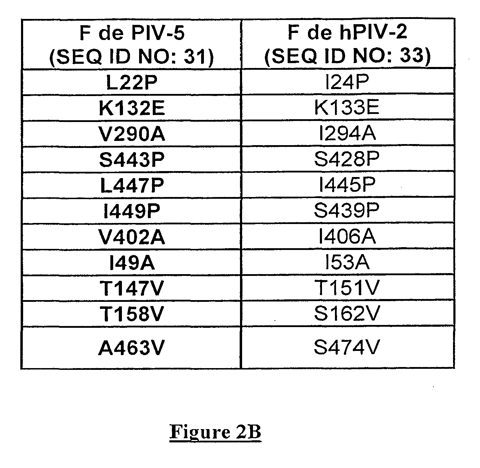

Les positions indiquées dans la protéine F de PIV-2 sont les positions qui correspondent à celles indiquées dans la protéine F de PIV-5 : *cf.* figure 2B, donnant le tableau des correspondances de positions.

La séquence de ladite protéine F de virus PIV-5 ou PIV-2 est telle que ci-dessus définie. Elle peut donc être notamment définie comme comprenant la séquence de SEQ ID NO: 34 (séquence consensus des protéines F de PIV-5 et PIV-2).

### Protéine mutante de protéine F de PIV-5 :

Selon un aspect de l'invention, une protéine mutante de l'invention comprend une séquence qui est susceptible de dériver de celle de la protéine F d'un virus PIV-5.

La séquence de ladite protéine F de PIV-5 est telle que ci-dessus définie. Elle peut notamment consister en :
- la séquence de SEQ ID NO: 31 (séquence de la protéine F de l'isolat WR de PIV-5 présentée en Figure 1A), ou de ladite séquence alternative « SEQ ID NO: 31 avec S en 443 », ou en
- une séquence variante de cette séquence de SEQ ID NO: 31 ou de ladite séquence alternative « SEQ ID NO: 31 avec S en 443 », cette séquence variante :
   o étant d'une taille identique à celle de SEQ ID NO: 31 (c'est-à-dire consistant en 529 acides aminés), ou étant d'une taille supérieure d'un maximum de 7 acides aminés à celle de SEQ ID NO: 31 (c'est-à-dire consistant en 530, 531, 532, 533, 534, 535 ou 536 acides aminés), ou étant d'une taille inférieure d'un maximum de 7 acides aminés à celle de SEQ ID NO: 31 (c'est-à-dire consistant en 522, 523, 524, 525, 526, 527 ou 528 acides aminés), et
   o présentant une identité de séquence de plus de 95%, préférentiellement d'au moins 96%, plus préférentiellement d'au moins 97%, par rapport à la séquence de SEQ ID NO: 31 ou à ladite séquence alternative « SEQ ID NO: 31 avec S en 443 », cette identité étant calculée sur la longueur de la séquence de SEQ ID NO: 31 ou (le cas échéant) de ladite séquence alternative « SEQ ID NO: 31 avec S en 443 ».

De préférence, la séquence de ladite protéine F de PIV-5 consiste en :
- la séquence de SEQ ID NO: 31 (séquence de la protéine F de l'isolat WR de PIV-5 présentée en Figure 1A), ou ladite séquence alternative « SEQ ID NO: 31 avec S en 443 », ou en
- une séquence variante de cette séquence de SEQ ID NO: 31 ou de ladite séquence alternative « SEQ ID NO: 31 avec S en 443 », cette séquence variante :
   o étant d'une taille identique à celle de SEQ ID NO: 31 (c'est-à-dire consistant en 529 acides aminés), ou étant d'une taille supérieure d'un maximum de 7 acides aminés à celle de SEQ ID NO: 31 (c'est-à-dire consistant en 530, 531, 532, 533, 534, 535 ou 536 acides aminés), et
   o présentant une identité de séquence de plus de 95%, préférentiellement d'au moins 96%, plus préférentiellement d'au moins 97%, par rapport à la séquence de SEQ ID NO: 31 ou à ladite séquence alternative « SEQ ID NO: 31 avec S en 443 », cette identité étant calculée sur la longueur de la séquence de SEQ ID NO: 31, ou, le cas échéant, de ladite séquence alternative « SEQ ID NO: 31 avec S en 443 ».

Des exemples de telles séquences variantes comprennent notamment la séquence de la protéine F d'un des isolats W3A, MIL, DEN, LN, MEL, Cryptovirus, CPI+, CPI-, H221, 78524, T1 et SER présentée en tableau 1 dans la présente demande (*cf.* ci-dessus), c'est-à-dire en l'une des séquences de SEQ ID NO: 35 à 46.

De préférence, la séquence de ladite protéine F de PIV-5 consiste en la séquence de SEQ ID NO: 31 (séquence de la protéine F de l'isolat WR de PIV-5 présentée en Figure 1A), ou en ladite séquence alternative « SEQ ID NO: 31 avec S en 443 », très préférentiellement en ladite séquence alternative « SEQ ID NO: 31 avec S en 443 ».

Ladite séquence susceptible de dériver de celle de la protéine F de PIV-5 peut ne pas comprendre de mutation autre que les mutations 22P, 132E, 290A, 449P ci-dessus mentionnées, par rapport à ladite séquence de protéine F de PIV-5. C'est notamment le cas de la séquence de SEQ ID NO: 65 (Fus8).

Alternativement, ladite séquence susceptible de dériver de celle de la protéine F de PIV-5 peut être susceptible de dériver de cette séquence de protéine F par lesdites mutations 22P, 132E, 290A, 449P ci-dessus mentionnées et par au moins une mutation autre que ces mutations 22P, 132E, 290A, 449P ci-dessus mentionnées, préférentiellement par :
- au moins une mutation de pré-fusion choisie parmi :
   - le remplacement de l'acide aminé en position 49 par l'acide aminé A,
   - le remplacement de l'acide aminé en position 402 par l'acide aminé A,
   - le remplacement de l'acide aminé en position 443 par l'acide aminé P,
   - le remplacement de l'acide aminé en position 447 par l'acide aminé P, et/ou par
- au moins une mutation de post-fusion choisie parmi :
   - le remplacement de l'acide aminé en position 147 par un acide aminé hydrophobe,
   - le remplacement de l'acide aminé en position 158 par un acide aminé hydrophobe.

De préférence, ladite séquence qui est susceptible de dériver de celle de ladite protéine F de PIV-5 est susceptible de dériver de cette séquence de protéine F par lesdites mutations 22P, 132E, 290A, 449P ci-dessus mentionnées, et par :
- au moins une mutation de pré-fusion choisie parmi :
   - le remplacement de l'acide aminé en position 49 par l'acide aminé A,
   - le remplacement de l'acide aminé en position 402 par l'acide aminé A, et/ou par
- au moins une mutation de post-fusion choisie parmi :
   - le remplacement de l'acide aminé en position 147 par un acide aminé hydrophobe,
   - le remplacement de l'acide aminé en position 158 par un acide aminé hydrophobe.

Ledit acide aminé hydrophobe est avantageusement choisi parmi V, I, L, préférentiellement V.

De préférence, ladite séquence qui est susceptible de dériver de celle de ladite protéine F de PIV-5 est susceptible de dériver de cette séquence de protéine F par lesdites mutations 22P, 132E, 290A, 449P ci-dessus mentionnées, et par :
- au moins une mutation de post-fusion choisie parmi :
   - le remplacement de l'acide aminé en position 147 par un acide aminé hydrophobe,
   - le remplacement de l'acide aminé en position 158 par un acide aminé hydrophobe ;
   et
- au moins une autre mutation de post-fusion, à savoir le remplacement de l'acide aminé en position 463 par un acide aminé hydrophobe, de préférence V, I ou L, plus préférentiellement V.

Une telle séquence susceptible de dériver de celle de ladite protéine F peut notamment être une séquence choisie parmi les séquences de SEQ ID NO: 67 à 79 (Fus10, Fus10.4, Fus10.5, Fus 11, Fus8.1, Fus8.2, Fus8.4, Fus8.5, Fus8.6, Fus8.7, Fus10.1, Fus10.2, Fus10.3, respectivement) ; *cf.* tableau 4 ci-dessous.

De préférence, ladite séquence qui est susceptible de dériver de celle de ladite protéine F de PIV-5 est susceptible de dériver de cette séquence de protéine F par lesdites mutations 22P, 132E, 290A, 449P ci-dessus mentionnées, et par au moins une mutation de post-fusion choisie parmi :
- le remplacement de l'acide aminé en position 147 par un acide aminé hydrophobe,
- le remplacement de l'acide aminé en position 158 par un acide aminé hydrophobe.

Une telle séquence susceptible de dériver de celle de ladite protéine F peut notamment être une séquence choisie parmi les séquences de SEQ ID NO: 68 à 79 (Fus10.4, Fus10.5, Fus 11, Fus8.1, Fus8.2, Fus8.4, Fus8.5, Fus8.6, Fus8.7, Fus10.1, Fus10.2, Fus10.3, respectivement) ; *cf.* tableau 4 ci-dessous.

De préférence, ladite séquence qui est susceptible de dériver de celle de ladite protéine F de PIV-5 est susceptible de dériver de cette séquence de protéine F par lesdites mutations 22P, 132E, 290A, 449P ci-dessus mentionnées, et par :
- au moins une mutation de pré-fusion choisie parmi :
   - le remplacement de l'acide aminé en position 49 par l'acide aminé A,
   - le remplacement de l'acide aminé en position 402 par l'acide aminé A,
   et par
- au moins une mutation de post-fusion choisie parmi :
   - le remplacement de l'acide aminé en position 147 par un acide aminé hydrophobe,
   - le remplacement de l'acide aminé en position 158 par un acide aminé hydrophobe.

Une telle séquence susceptible de dériver de celle de ladite protéine F peut notamment être une séquence choisie parmi les séquences de SEQ ID NO: 68, 69, 70 (Fus10.4, Fus10.5, Fus 11, respectivement) ; *cf.* tableau 4 ci-dessous.

De préférence, ladite séquence qui est susceptible de dériver de celle de ladite protéine F de PIV-5 est susceptible de dériver de cette séquence de protéine F par lesdites mutations 22P, 132E, 290A, 449P ci-dessus mentionnées, et par les deux mutations de post-fusion, c'est-à-dire par :
- le remplacement de l'acide aminé en position 147 par un acide aminé hydrophobe,
- le remplacement de l'acide aminé en position 158 par un acide aminé hydrophobe.

Une telle séquence susceptible de dériver de celle de ladite protéine F peut notamment être une séquence choisie parmi les séquences de SEQ ID NO : 76, 78 (Fus8.7, Fus10.2, respectivement) ; *cf.* tableau 4 ci-dessous.

**Tableau 4 : sélection de protéines mutantes de l'invention (protéines mutantes de protéine F de PIV-5)**

| **N° de Fus** | **Score de fusion *(cf.*** **Figure 6B****)** | **SEQ ID NO:** | **Mutation(s) additionnelle(s) en sus des trois mutations d'autonomie (22P, 132E, 290A) et de la mutation de pré-fusion 449P** | |
|---|---|---|---|---|
| | | | Pré-fusion | Post-fusion |
| 8 | Environ 7 | 65 | - | - |
| 10 | Environ 6 | 67 | 49A | - |
| 10.4 | Environ 7 | 68 | 402A | 147V, 158V, 463V |
| 10.5 | Environ 7,5 | 69 | 49A, 402A | 147V, 158V, 463V |
| 11 | Environ 7,5 | 70 | 402A | - |
| 8.1 | Environ 6,5 | 71 | - | 147V |
| 8.2 | Environ 6,5 | 72 | - | 158V |
| 8.4 | Environ 7,5 | 73 | | 147V, 158V |
| 8.5 | Environ 6,5 | 74 | | 147V,463V |
| 8.6 | Environ 6 | 75 | | 158V,463V |
| 8.7 | Environ 9 | 76 | | 147V, 158V, 463V |
| 10.1 | Environ 5 | 77 | | 158V |
| 10.2 | Environ 7 | 78 | | 147V, 158V |
| 10.3 | Environ 6,5 | 79 | | 147V, 158V, 463V |

Les mutations indiquées dans le tableau 4 peuvent être introduites dans la protéine F de n'importe quel isolat PIV-5, c'est-à-dire de l'isolat WR ou d'un isolat variant.

Elles peuvent donc plus particulièrement être introduites dans la séquence de protéine F de SEQ ID NO: 31 présentée en Figure 1A (protéine F de WR disponible dans la base de données Genbank sous le numéro AB021962).

Comme indiqué ci-dessus, l'échantillon d'isolat WR que les inventeurs ont reçu de l'ATCC et qu'ils ont utilisé pour la construction et la production des protéines mutantes décrites dans les exemples ci-dessous ne présentait toutefois pas l'acide aminé P en position 443 de la protéine F (contrairement à ce qui était attendu au vu de la séquence disponible dans Genbank), mais l'acide aminé S. Les mutations indiquées dans le tableau 4 peuvent donc être introduites dans ladite séquence alternative « SEQ ID NO: 31 avec S en 443 » (SEQ ID NO: 65, 67 à 79).

### Protéine mutante de protéine F de PIV-2 :

Selon un autre aspect de l'invention, une protéine mutante de l'invention comprend une séquence qui est susceptible de dériver de celle de la protéine F d'un virus PIV-2.

La séquence de ladite protéine F de PIV-2 est telle que ci-dessus définie. Elle peut notamment consister en :
- la séquence de SEQ ID NO: 33 (séquence de la protéine F de l'isolat Greer de PIV-2 présentée en Figure 1B), ou en
- une séquence variante de cette séquence de SEQ ID NO: 33, cette séquence variante :
   o étant d'une taille identique à celle de SEQ ID NO: 33 (c'est-à-dire consistant en 551 acides aminés), ou étant d'une taille supérieure d'un maximum de 2 acides aminés à celle de SEQ ID NO: 33 (c'est-à-dire consistant en 552 ou 553 acides aminés), ou étant d'une taille inférieure d'un maximum de 2 acides aminés à celle de SEQ ID NO: 33 (c'est-à-dire consistant en 549 ou 550 acides aminés), et
   o présentant une identité de séquence de plus de 95%, préférentiellement d'au moins 96%, plus préférentiellement d'au moins 97%, par rapport à la séquence de SEQ ID NO: 33, cette identité étant calculée sur la longueur de la séquence de SEQ ID NO: 33.

De préférence, la séquence de ladite protéine F de PIV-2 consiste en :
- la séquence de SEQ ID NO: 33 (séquence de la protéine F de l'isolat Greer de PIV-2 présentée en Figure 1B), ou en
- une séquence variante de cette séquence de SEQ ID NO: 33, cette séquence variante :
   o étant d'une taille identique à celle de SEQ ID NO: 33 (c'est-à-dire consistant en 551 acides aminés), ou étant d'une taille supérieure d'un maximum de 2 acides aminés à celle de SEQ ID NO: 33 (c'est-à-dire consistant en 552 ou 553 acides aminés), et
   o présentant une identité de séquence de plus de 95%, préférentiellement d'au moins 96%, plus préférentiellement d'au moins 97%, par rapport à la séquence de SEQ ID NO: 33, cette identité étant calculée sur la longueur de la séquence de SEQ ID NO: 33.

Plus préférentiellement, la séquence de ladite protéine F de PIV-2 consiste en :
- la séquence de SEQ ID NO: 33 (séquence de la protéine F de l'isolat Greer de PIV-2 présentée en Figure 1B), ou en
- une séquence variante de cette séquence de SEQ ID NO: 33, cette séquence variante :
   o étant d'une taille identique à celle de SEQ ID NO: 33 (c'est-à-dire consistant en 551 acides aminés), et
   o présentant une identité de séquence de plus de 95%, préférentiellement d'au moins 96%, plus préférentiellement d'au moins 97%, par rapport à la séquence de SEQ ID NO: 33, cette identité étant calculée sur la longueur de la séquence de SEQ ID NO: 33.

Très préférentiellement, la séquence de ladite protéine F consiste en la séquence de SEQ ID NO: 33 (séquence de la protéine F de l'isolat Greer de PIV-2 présentée en Figure 1B).

Ladite séquence susceptible de dériver de celle de la protéine F de PIV-2 peut ne pas comprendre de mutation autre que les mutations 24P, 133E, 294A, 439P ci-dessus mentionnées, par rapport à ladite séquence de protéine F de PIV-2. C'est par exemple le cas de la protéine mutante comprenant ou consistant en la séquence de SEQ ID NO: 90.

Alternativement, ladite séquence susceptible de dériver de celle de la protéine F de PIV-2 peut être susceptible de dériver de cette séquence de protéine F par lesdites mutations 24P, 133E, 294A, 439P ci-dessus mentionnées et par au moins une mutation autre que ces mutations 24P, 133E, 294A, 439P ci-dessus mentionnées, préférentiellement par :
- au moins une mutation de pré-fusion choisie parmi :
   - le remplacement de l'acide aminé en position 53 par l'acide aminé A,
   - le remplacement de l'acide aminé en position 406 par l'acide aminé A,
   - le remplacement de l'acide aminé en position 428 par l'acide aminé P,
   - le remplacement de l'acide aminé en position 445 par l'acide aminé P,
   et/ou par
- au moins une mutation de post-fusion choisie parmi :
   - le remplacement de l'acide aminé en position 151 par un acide aminé hydrophobe,
   - le remplacement de l'acide aminé en position 162 par un acide aminé hydrophobe.

C'est par exemple le cas de la protéine mutante comprenant l'une des séquences de SEQ ID NO: 91 à 103 (ou consistant en l'une de ces séquences).

De préférence, ladite séquence qui est susceptible de dériver de celle de ladite protéine F dérive de cette séquence de protéine F par lesdites mutations 24P, 133E, 294A, 439P ci-dessus mentionnées et par :
- au moins une mutation de pré-fusion choisie parmi :
   - le remplacement de l'acide aminé en position 53 par l'acide aminé A,
   - le remplacement de l'acide aminé en position 406 par l'acide aminé A,
   et/ou par
- au moins une mutation de post-fusion choisie parmi :
   - le remplacement de l'acide aminé en position 151 par un acide aminé hydrophobe,
   - le remplacement de l'acide aminé en position 162 par un acide aminé hydrophobe.

C'est par exemple le cas de la protéine mutante comprenant l'une des séquences de SEQ ID NO: 91 à 103 (ou consistant en l'une de ces séquences).

Avantageusement, ledit acide aminé hydrophobe est choisi parmi V, I, L, préférentiellement V.

De préférence, ladite séquence qui est susceptible de dériver de celle de ladite protéine F dérive de cette séquence de protéine F par lesdites mutations 24P, 133E, 294A, 439P ci-dessus mentionnées et par :
- au moins une mutation de post-fusion choisie parmi :
   - le remplacement de l'acide aminé en position 151 par un acide aminé hydrophobe,
   - le remplacement de l'acide aminé en position 162 par un acide aminé hydrophobe ;
   et
- au moins une autre mutation de post-fusion, à savoir le remplacement de l'acide aminé en position 474 par un acide aminé hydrophobe, de préférence V, I ou L, plus préférentiellement V.

C'est par exemple le cas de la protéine mutante comprenant l'une des séquences de SEQ ID NO: 92, 93, 98, 99, 100, 103 (ou consistant en l'une de ces séquences).

De préférence, ladite séquence qui est susceptible de dériver de celle de ladite protéine F dérive de cette séquence de protéine F par lesdites mutations 24P, 133E, 294A, 439P ci-dessus mentionnées et par au moins une mutation de post-fusion choisie parmi :
- le remplacement de l'acide aminé en position 151 par un acide aminé hydrophobe,
- le remplacement de l'acide aminé en position 162 par un acide aminé hydrophobe.

C'est par exemple le cas de la protéine mutante comprenant l'une des séquences de SEQ ID NO: 92, 93 et 94 à 103 (ou consistant en l'une de ces séquences).

De préférence, ladite séquence qui est susceptible de dériver de celle de ladite protéine F dérive de cette séquence de protéine F par lesdites mutations 24P, 133E, 294A, 439P ci-dessus mentionnées et par :
- au moins une mutation de pré-fusion choisie parmi :
   - le remplacement de l'acide aminé en position 53 par l'acide aminé A,
   - le remplacement de l'acide aminé en position 406 par l'acide aminé A,
   et par
- au moins une mutation de post-fusion choisie parmi :
   - le remplacement de l'acide aminé en position 151 par un acide aminé hydrophobe,
   - le remplacement de l'acide aminé en position 162 par un acide aminé hydrophobe.

C'est par exemple le cas de la protéine mutante comprenant l'une des séquences de SEQ ID NO: 92, 93 (ou consistant en l'une de ces séquences).

De préférence, ladite séquence qui est susceptible de dériver de celle de ladite protéine F dérive de cette séquence de protéine F par lesdites mutations 24P, 133E, 294A, 439P ci-dessus mentionnées et par les deux mutations de post-fusion, c'est-à-dire par :
- le remplacement de l'acide aminé en position 151 par un acide aminé hydrophobe,
- le remplacement de l'acide aminé en position 162 par un acide aminé hydrophobe.

C'est par exemple le cas de la protéine mutante comprenant l'une des séquences de SEQ ID NO: 92, 93, 97, 100, 102, 103 (ou consistant en l'une de ces séquences).

La présente demande vise plus particulièrement les protéines mutantes de protéine F de PIV-2 qui correspondent à celles présentées en tableau 4 ci-dessus pour PIV-5, en tenant compte que, comme indiqué en Figure 2B :
- la mutation 49A de F PIV-5 correspond à la mutation 53A dans F PIV-2,
- la mutation 402A de F PIV-5 correspond à 406A dans F PIV-2,
- la mutation 147V de F PIV-5 correspond à 151V dans F HIV-2,
- la mutation 158V de F PIV-5 correspond à 162V dans F PIV-2,
- la mutation 463V de F PIV-5 correspond à 474V dans F PIV-2.

**Tableau 5 : sélection de protéines mutantes de l'invention (protéines mutantes de protéine F de PIV-2)**

| **SEQ ID NO:** | **Mutation(s) additionnelle(s) en sus des trois mutations d'autonomie (24P, 133E, 294A) et de la mutation de pré-fusion 439P** | |
|---|---|---|
| | Pré-fusion | Post-fusion |
| 90 | - | - |
| 91 | 53A | - |
| 92 | 406A | 151V, 162V, 474V |
| 93 | 53A, 406A | 151V, 162V, 474V |
| 94 | 406A | - |
| 95 | - | 151V |
| 96 | - | 162V |
| 97 | | 151V, 162V |
| 98 | | 151V, 474V |
| 99 | | 162V, 474V |
| 100 | | 151V, 162V, 474V |
| 101 | | 162V |
| 102 | | 151V, 162V |
| 103 | | 151V, 162V, 474V |

Les mutations indiquées dans le tableau 5 peuvent être introduites dans la protéine F de n'importe quel isolat PIV-2, c'est-à-dire de l'isolat Greer ou d'un isolat variant. Elles peuvent donc plus particulièrement être introduites dans la séquence de protéine F de SEQ ID NO: 33 présentée en Figure 1B (protéine F de Greer ; SEQ ID NO: 90 à 103).

### Site de clivage :

Conformément à la présente invention, une protéine mutante de l'invention peut comprendre une séquence qui est susceptible de dériver de celle de la protéine F d'un virus PIV-5 ou PIV-2 par :
- les mutations ci-dessus mentionnées, et en outre par
- substitution du site de clivage natif de ladite protéine F par un autre site de clivage enzymatique, et/ou par insertion dans ladite protéine F d'un site de clivage enzymatique autre que le site de clivage natif de cette protéine F, de préférence par substitution du site de clivage natif de ladite protéine F par un autre site de clivage enzymatique.

Le site de clivage d'une protéine F de PIV-5 ou PIV-2 est le site de clivage des deux sous-unités (FI et F2) de cette protéine F ; *cf.* Figure 9 pour une illustration sur la protéine F de PIV-5.

Dans la forme native de la protéine F de PIV-5 et PIV-2, ce site de clivage est un site clivé par des furines.

Dans la forme native de la protéine F de PIV-5, ce site de clivage consiste en la séquence RRRRR (SEQ ID NO: 23). Il se situe en positions 98 à 102 de la forme native de la protéine F de PIV-5 (*cf.* Figure 1A ; *cf.* Figure 9). Un exemple de fragment de séquence de protéine F de PIV-5 comprenant le site de clivage natif (ou naturel) de la protéine F de PIV-5 est : IGENLETIRNQLIPT**RRRRR**FAGVVIGL (SEQ ID NO: 24).

Dans la forme native de la protéine F de PIV-2, le site de clivage consiste en la séquence KTRQKR (SEQ ID NO: 25). Il se situe en positions 101 à 106 de la forme native de la protéine F de PIV-2 (*cf.* Figure 1B). Un exemple de fragment de séquence de protéine F de PIV-2 comprenant le site de clivage natif (ou naturel) de la protéine F de PIV-2 est LTPLIENLSKISTVTDT**KTRQKR**FAGVVVGLAALGVA (SEQ ID NO: 26).

De préférence, ledit site de clivage autre que le site de clivage natif est un site de clivage tissu-spécifique.

De préférence, ledit site de clivage autre que le site de clivage natif est un site de clivage pour une enzyme exprimée spécifiquement par un ou des tissus tumoraux, très préférentiellement un site de clivage pour une enzyme exprimée spécifiquement par un ou des tissus métastasiques.

Par exemple, ledit site de clivage autre que le site de clivage natif peut être un site de clivage pour une métallo-protéase, tel que le site de clivage pour la métallo-protéase matricielle 9 (MMP-9) ; *cf.* exemple 2 ci-dessous.

Un site de clivage pour la métallo-protéase matricielle 9 (MMP-9) peut comprendre ou consister en la séquence PXXHyS/T (SEQ ID NO: 27) avec X = n'importe quel acide aminé, et avec Hy = n'importe quel acide aminé hydrophobe (c'est-à-dire n'importe quel acide aminé parmi F, M, V, L, I).

Par exemple, un site de clivage pour la métallo-protéase matricielle 9 (MMP-9) peut comprendre ou consister en la séquence PRRIT (SEQ ID NO: 28) et/ou la séquence IGENLETIRNQLIPT**PRRIT**FAGVVIGL (SEQ ID NO: 29).

Des exemples de protéines mutantes de l'invention comprenant un tel site de clivage (par substitution du site de clivage natif) comprennent les protéines mutantes dont la séquence susceptible de dériver de la protéine F (de PIV-5) est la séquence de SEQ ID NO: 88 ou 89 (Fus8M et Fus8.7M, respectivement ; *cf.* exemple 2 ci-dessous).

### Acides nucléiques de l'invention:

La présente demande est également relative à un acide nucléique, ADN ou ARN, qui code une protéine mutante selon l'invention (selon le code génétique universel et en tenant compte de la dégénérescence de ce code), et à un acide nucléique complémentaire d'un tel acide nucléique (acide nucléique de même longueur parfaitement complémentaire).

De tels acides nucléiques dérivent de la séquence de SEQ ID NO: 30 (séquence codant la protéine F de PIV-5 native), ou d'une séquence alternative codant ladite séquence alternative « SEQ ID NO: 31 avec S en 443 », ou d'une séquence variante codant une protéine F variante, ou bien de la séquence de SEQ ID NO: 32 (séquence codant la protéine F de PIV-2 native) ou d'une séquence variante codant une protéine F variante.

### Vecteurs de l'invention :

La présente demande est également relative à un vecteur d'acide nucléique, plus particulièrement à un vecteur de transfection, transduction ou transformation, comprenant au moins un acide nucléique selon l'invention.

Un tel vecteur peut avantageusement être un vecteur d'expression.

De préférence, il s'agit d'un vecteur permettant l'expression dudit au moins un acide nucléique dans une cellule animale (cellule animale non-humaine et/ou cellule humaine), plus préférentiellement :
- dans une cellule humaine, avantageusement dans une cellule humaine pathologique, plus particulièrement une cellule humaine tumorale, plus préférentiellement une cellule de mélanome métastasique, ou
- dans une cellule placentaire.

Un tel vecteur d'expression peut avantageusement être un vecteur adénoviral.

Ce vecteur adénoviral peut comprendre des éléments de régulation de l'expression dudit acide nucléique, préférentiellement un promoteur, permettant une expression dudit acide nucléique dans des cellules tumorales, de préférence dans des cellules métastasiques, plus préférentiellement dans des cellules de mélanome métastasiques.

De préférence, cette expression est spécifique. Avantageusement, cette expression est suffisamment spécifique pour permettre l'expression dudit acide nucléique dans lesdites cellules tumorales ou métastasiques, sans qu'il y ait pour autant expression significative dans des cellules non-tumorales (voire, non-métastasiques).

Avantageusement, un tel vecteur adénoviral est un vecteur adénoviral oncolytique.

Alternativement, un vecteur d'expression de l'invention peut être un vecteur adénoviral qui comprend des éléments de régulation de l'expression dudit acide nucléique, préférentiellement un promoteur, permettant une expression dudit acide nucléique dans des cellules placentaires, de préférence dans des cellules placentaires pathologiques qui présentent une insuffisance de fusogénicité.

De préférence, cette expression est spécifique. Avantageusement, cette expression est suffisamment spécifique pour permettre l'expression dudit acide nucléique dans lesdites cellules placentaires, sans qu'il y ait pour autant expression significative dans des cellules non-placentaires.

Un vecteur de l'invention peut alternativement ou complémentairement être un vecteur permettant l'insertion dudit au moins un acide nucléique dans le génome d'une cellule animale (cellule animale non-humaine et/ou cellule humaine), plus préférentiellement d'une cellule humaine, avantageusement d'une cellule humaine pathologique, plus particulièrement d'une cellule humaine tumorale, préférentiellement une cellule humaine métastasique, plus préférentiellement dans des cellules de mélanome métastasiques. Un tel vecteur est plus particulièrement destiné à la thérapie génique des tumeurs, particulièrement des tumeurs métastasiques, plus particulièrement des mélanomes métastasiques.

La présente demande est également relative à un vecteur qui comprend au moins un acide nucléique de l'invention, et qui permet l'insertion dudit au moins un acide nucléique dans le génome d'une cellule animale (cellule animale non-humaine et/ou cellule humaine), plus préférentiellement d'une cellule humaine, avantageusement d'une cellule placentaire, préférentiellement une cellule placentaire humaine. Un tel vecteur est plus particulièrement destiné à la thérapie génique des maladies ou états impliquant une déficience du développement placentaire.

### Cellules de l'invention :

La présente demande est également relative à une cellule, qui comprend au moins une protéine mutante selon l'invention, et/ou au moins un acide nucléique, ADN ou ARN selon l'invention, et/ou au moins un vecteur selon l'invention.

Une telle cellule peut être une cellule humaine ou une cellule animale non-humaine.

De préférence, une telle cellule est une cellule tumorale, préférentiellement une cellule métastasique, plus préférentiellement une cellule de mélanome métastasique.

Une telle cellule trouve notamment des applications en tant que cellule à capacité fusogène capable d'induire la formation de syncytia, tel que ci-dessous décrit.

Alternativement, une cellule de l'invention peut être une cellule non-tumorale du système immunitaire humain ou animal non-humain, préférentiellement une cellule dendritique non-tumorale humaine ou animale non-humaine, ladite cellule exprimant à sa surface au moins une protéine mutante selon l'invention. Une telle cellule trouve notamment des applications en tant qu'agent capable d'induire la production d'inhibiteur de la fusion cellulaire, par exemple par immunisation active, tel que ci-dessous décrit.

### Applications médicales (pro-fusion):

Une protéine mutante de l'invention, et/ou un acide nucléique, ADN ou ARN de l'invention, et/ou un vecteur de l'invention, et/ou une cellule de l'invention, peuvent être utilisés dans le traitement et/ou la prévention et/ou la palliation d'une maladie ou d'un état qui implique la présence et/ou la prolifération de cellules pathologiques et/ou non favorables à l'état de santé de l'organisme, plus particulièrement dans le traitement et/ou la prévention et/ou la palliation d'une maladie ou d'un état qui implique une insuffisance de fusogénicité cellulaire, préférentiellement dans le traitement et/ou la prévention et/ou la palliation d'une maladie ou d'un état néoplasique, telle qu'une tumeur, une tumeur métastasique, avantageusement un mélanome métastasique.

De telles maladies ou états peuvent être traités et/ou prévenus et/ou palliés par diminution ou suppression de ces cellules pathologiques et/ou non favorables.

Une protéine mutante de l'invention exprimée à la surface de telles cellules induira la fusion de ces cellules, et par suite la formation de syncytia, conduisant à la destruction (ou à tout le moins à la diminution du nombre) de ces cellules.

Une protéine mutante de l'invention, et/ou un acide nucléique, ADN ou ARN de l'invention, et/ou un vecteur de l'invention, et/ou une cellule de l'invention, peuvent être utilisés dans le traitement et/ou la prévention et/ou la palliation d'une maladie ou d'un état qui implique une déficience du développement placentaire.

De telles maladies ou états peuvent être traités et/ou prévenus et/ou palliés par induction ou stimulation de la fusion cellulaire placentaire.

La présente demande est donc également relative à une composition pharmaceutique ou un médicament qui comprend au moins une protéine mutante de l'invention et/ou au moins un acide nucléique, ADN, ARN de l'invention, et/ou au moins un vecteur de l'invention et/ou une cellule de l'invention.

Une telle composition pharmaceutique ou un tel médicament peut notamment être destiné au traitement et/ou la prévention et/ou la palliation d'une maladie ou d'un état qui implique la présence et/ou la prolifération de cellules pathologiques et/ou non favorables à l'état de santé de l'organisme, telle que ci-dessus indiquée (par exemple, tumeur, tumeur métastasique, mélanome métastasique), ou au traitement et/ou la prévention et/ou la palliation d'une maladie ou d'un état qui implique une insuffisance de fusogénicité cellulaire (par exemple, déficience du développement placentaire).

Une telle composition pharmaceutique ou un tel médicament peut en outre comprendre au moins un véhicule pharmaceutiquement et/ou physiologiquement acceptable.

Une protéine mutante de l'invention (ou un acide nucléique, ADN, ARN, ou un vecteur d'expression de l'invention), peut être utilisée pour être exprimée par une cellule humaine ou une cellule animale non-humaine, préférentiellement pour être exprimée à la surface d'une telle cellule.

Cette cellule peut être une cellule pathologique, préférentiellement une cellule tumorale, plus préférentiellement une cellule métastasique, plus préférentiellement une cellule de mélanome métastasique, ou peut être une cellule non-tumorale, par exemple une cellule saine.

Plus particulièrement, des cellules pathologiques qui ont été prélevées sur un patient humain ou sur un sujet animal non-humain malade, peuvent être traitées *ex vivo* (ou *in vitro*) par mise en contact avec au moins une protéine mutante de l'invention et/ou au moins un acide nucléique, ADN ou ARN de l'invention et/ou au moins un vecteur d'expression de l'invention de sorte à leur faire exprimer une protéine mutante de l'invention.

Alternativement ou complémentairement, des cellules non-pathologiques mais localisées à proximité des cellules pathologiques du patient ou sujet peuvent être prélevées pour subir ce traitement.

Les cellules ainsi traitées *ex vivo* (ou *in vitro*) peuvent alors être destinées à être ré-administrées audit patient ou sujet.

De telles cellules sont utiles pour le traitement et/ou la prévention et/ou la palliation de la pathologie dont est affecté ledit patient ou sujet, par exemple une tumeur, une tumeur métastasique, un mélanome métastasique.

Alternativement, cette cellule peut être une cellule placentaire, plus particulièrement une cellule placentaire souffrant d'une insuffisance de fusogénicité. Une fois traitée par expression d'une protéine mutante de l'invention à sa surface, une telle cellule peut être destinée au traitement et/ou la prévention et/ou la palliation d'une déficience du développement placentaire.

La présente demande est donc plus particulièrement relative à une protéine mutante selon l'invention, un acide nucléique, ADN ou ARN selon l'invention, un vecteur selon l'invention, une cellule selon l'invention, pour une utilisation dans le traitement et/ou la prévention et/ou la palliation d'une maladie ou d'un état néoplasique, préférentiellement d'une tumeur plus préférentiellement d'une tumeur métastasique, très préférentiellement d'un mélanome métastasique.

La présente demande est également plus particulièrement relative à une protéine mutante selon l'invention, un acide nucléique, ADN ou ARN selon l'invention, un vecteur selon l'invention, une cellule selon l'invention, pour une utilisation dans le traitement et/ou la prévention et/ou la palliation d'une déficience du développement placentaire.

### Inhibiteurs de fusion selon l'invention :

La présente demande est également relative à des produits qui ont la capacité de diminuer ou bloquer la fusion cellulaire. Ces produits sont des inhibiteurs d'une ou plusieurs des protéines mutantes de l'invention.

De tels inhibiteurs peuvent être utilisés dans le traitement et/ou la prévention et/ou la palliation d'une maladie ou d'un état qui implique un excès de fusogénicité cellulaire, préférentiellement dans le traitement et/ou la prévention et/ou la palliation d'une infection à virus enveloppé (telle qu'une infection à VIH, *Influenza, Parainfluenza, Rhabdovirus*), d'une allergie, d'une maladie auto-immune, d'un rejet de greffe.

De préférence, un inhibiteur selon l'invention est :
- un anticorps dirigé contre une protéine mutante selon l'invention, ou un fragment Fab ou F(ab')2 d'un tel anticorps, ou
- un acide nucléique aptamère ou un peptide aptamère qui se lie spécifiquement à au moins une protéine mutante selon l'invention, ou à un acide nucléique, ADN, ARN selon l'invention, ou
- une cellule du système immunitaire recombinante, préférentiellement une cellule dendritique recombinante, qui exprime à sa surface au moins une protéine mutante selon l'invention, ou
- un acide nucléique anti-sens d'un acide nucléique selon l'invention, ou
- un petit ARN interférant (*small interfering RNA ; siRNA*) comprenant un ARN double-brin de 19 à 22 nucléotides, capable de se lier (de s'hybrider) à un acide nucléique selon l'invention.

La présente demande est donc également relative à une cellule non-tumorale du système immunitaire humain ou animal non-humain, préférentiellement une cellule dendritique humaine ou animale non-humaine, ladite cellule exprimant à sa surface au moins une protéine mutante selon l'invention, ainsi qu'à l'utilisation de cette cellule dans le traitement et/ou la prévention et/ou la palliation d'une maladie ou d'un état qui implique un excès de fusogénicité cellulaire, préférentiellement dans le traitement et/ou la prévention et/ou la palliation d'une infection à virus enveloppé (telle qu'une infection à VIH, *Influenza*, *Parainfluenza, Rhabdovirus*), d'une allergie, d'une maladie auto-immune, d'un rejet de greffe.

La présente demande est également relative à un anticorps dirigé contre une protéine mutante selon l'invention, ou contre plusieurs des protéines mutantes de l'invention. De préférence, cet anticorps est un anticorps spécifique de ladite ou desdites protéines mutantes de l'invention. Avantageusement, cet anticorps est un anticorps monoclonal. Un inhibiteur de l'invention peut être un fragment conservateur d'un tel anticorps, tel qu'un fragment Fab ou F(ab')2.

Un tel anticorps ou fragment d'anticorps peut être destiné à bloquer ou inhiber un mécanisme de fusion cellulaire, par exemple en administrant cet anticorps ou fragment d'anticorps à un patient ou sujet qui en a le besoin.

Alternativement, ou complémentairement, une protéine mutante de l'invention peut être destinée à être elle-même administrée audit patient ou sujet de sorte à induire une immunisation active contre cette protéine, c'est-à-dire de sorte à induire la production par ledit patient ou sujet d'anticorps anti-protéine mutante. Si nécessaire ou souhaité, un ou plusieurs adjuvants de vaccin peuvent être administrés de manière conjointe ou différée dans le temps avec cette ou ces protéines mutantes.

La présente demande est donc également relative à un vaccin thérapeutique et/ou préventif et/ou palliatif, qui comprend au moins une protéine mutante de l'invention à titre d'agent immunogène, et avantageusement au moins un adjuvant d'immunisation. Un tel vaccin peut être, destiné au traitement et/ou à la prévention et/ou à la palliation d'une maladie ou d'un état qui implique un excès de fusogénicité cellulaire, préférentiellement dans le traitement et/ou la prévention et/ou la palliation d'une infection à virus enveloppé (telle qu'une infection à VIH, *Influenza*, *Parainfluenza*, *Rhabdovirus*), d'une allergie, d'une maladie auto-immune, d'un rejet de greffe.

La présente demande est également relative à :
- un acide nucléique aptamère ou un peptide aptamère qui se lie spécifiquement à au moins une protéine mutante de l'invention, ou à un acide nucléique, ADN, ARN de l'invention,
- une cellule du système immunitaire recombinante, préférentiellement une cellule dendritique recombinante, qui exprime à sa surface au moins une protéine mutante de l'invention,
- un acide nucléique anti-sens d'un acide nucléique de l'invention,
- un petit ARN interférant (*small interfering RNA* ; *siRNA*) comprenant un ARN double-brin de 19 à 22 nucléotides, capable de se lier (de s'hybrider) à un acide nucléique de l'invention, et avantageusement de bloquer ou inhiber la transcription de cet acide nucléique.

De tels produits sont également des inhibiteurs de l'invention. Ils peuvent donc être destinés au traitement et/ou à la prévention et/ou à la palliation d'une maladie ou d'un état qui implique un excès de fusogénicité cellulaire, tel que ci-dessus indiqué. Ils sont plus particulièrement destinés au traitement et/ou à la prévention et/ou à la palliation d'une maladie ou d'un état qui implique au moins un gène expresseur ou hyper-expresseur de protéine F.

La présente demande est donc également relative à une composition pharmaceutique ou un médicament qui comprend au moins un inhibiteur de l'invention.

Une telle composition pharmaceutique ou un tel médicament peut notamment être destiné au traitement et/ou la prévention et/ou la palliation d'une maladie ou d'un état qui implique un excès de fusogénicité cellulaire, tel que ci-dessus indiqué.

Une telle composition pharmaceutique ou un tel médicament peut en outre comprendre au moins un véhicule pharmaceutiquement et/ou physiologiquement acceptable.

La présente demande est plus particulièrement relative à un inhibiteur selon l'invention, pour une utilisation dans le traitement et/ou la prévention et/ou la palliation d'une maladie ou d'un état impliquant un excès de fusogénicité cellulaire, ladite maladie ou état étant une infection à virus enveloppé (préférentiellement une infection à VIH et/ou *Influenza* et/ou *Parainfluenza* et/ou *Rhabdovirus*), une allergie, une maladie auto-immune, un rejet de greffe.

La présente demande est plus particulièrement relative à une protéine mutante selon l'invention, pour une utilisation à titre d'agent immunogène dans le traitement et/ou la prévention et/ou la palliation d'une maladie ou d'un état impliquant un excès de fusogénicité cellulaire, ladite maladie ou d'un état étant une infection à virus enveloppé (préférentiellement une infection à VIH et/ou *Influenza* et/ou *Parainfluenza* et/ou *Rhabdovirus*), une allergie, une maladie auto-immune, un rejet de greffe.

La présente demande est plus particulièrement relative à un vaccin ou composition vaccinale, plus particulièrement un vaccin ou composition vaccinale destiné au traitement et/ou à la prévention et/ou à la palliation d'une maladie ou d'un état impliquant un excès de fusogénicité cellulaire, ladite maladie ou état étant une infection à virus enveloppé (préférentiellement une infection à VIH et/ou *Influenza* et/ou *Parainfluenza* et/ou *Rhabdovirus*), une allergie, une maladie auto-immune, un rejet de greffe. Un tel vaccin ou composition vaccinale comprend au moins protéine mutante selon l'invention, et optionnellement au moins un adjuvant physiologiquement acceptable.

### Applications diagnostiques et pronostiques :

La présente demande est également relative à une méthode, plus particulièrement une méthode *in vitro*, pour le diagnostic ou le pronostic d'une maladie ou d'un état impliquant :
- une formation insuffisante de syncytia, telle qu'une tumeur, une tumeur métastasique, un mélanome métastasique ou une déficience du développement placentaire, ou au contraire
- une formation excessive de syncytia, telle qu'une infection à virus enveloppé (préférentiellement une infection à VIH et/ou *Influenza* et/ou *Parainfluenza* et/ou *Rhabdovirus*), une allergie, une maladie auto-immune, un rejet de greffe.

La méthode de diagnostic ou pronostic de l'invention comprend la détection d'au moins une protéine mutante selon l'invention ou d'au moins un acide nucléique selon l'invention, par exemple dans un échantillon biologique tel qu'un échantillon biologique qui a été prélevé sur le patient ou sujet objet dudit diagnostic ou pronostic.

Cette détection peut par exemple être réalisée par séquençage des protéines ou acides nculéiques contenus dans ledit échantillon.

Cette détection peut par exemple être réalisée par détection de ladite au moins une protéine mutante de l'invention à l'aide d'un anticorps, d'un peptide aptamère, ou d'un oligonucléotide aptamère se liant à ladite au moins une protéine mutante, plus particulièrement à l'aide d'un anticorps, peptide aptamère, ou oligonucléotide aptamère de l'invention.

Cette détection peut par exemple être réalisée par détection dudit au moins un acide nucléique de l'invention à l'aide d'un acide nucléique, d'un peptide aptamère, ou d'un oligonucléotide aptamère se liant audit au moins un acide nucléique, plus particulièrement à l'aide d'un acide nucléique complémentaire d'un acide nucléique de l'invention, d'un peptide aptamère, ou d'un oligonucléotide aptamère de l'invention.

La présente demande est également relative audit anticorps, peptide aptamère, oligonucléotide aptamère, acide nucléique complémentaire, pour leur utilisation dans une méthode de diagnostic ou pronostic d'une formation insuffisante, ou au contraire excessive de syncytia.

### Applications biotechnologiques (criblage) :

La présente demande est également relative à une méthode, plus particulièrement une méthode *in vitro*, pour le criblage d'un composé capable de diminuer ou bloquer la formation de syncytia. La méthode de l'invention comprend la mise en contact d'un composé candidat avec des cellules exprimant au moins une protéine mutante selon l'invention, de sorte à déterminer si ce composé candidat diminue ou bloque la fusion desdites cellules (par exemple, en comparant le niveau de fusion atteint en présence dudit composé candidat à celui atteint en son absence).

De tels composés sont des principes actifs candidats pour le traitement et/ou la prévention et/ou la palliation d'une maladie ou d'un état impliquant un excès de fusogénicité cellulaire, telles que les infections à virus enveloppés, les allergies, les maladies auto-immunes, les rejets de greffe.

### Applications biotechnologiques (myélome, hybridome) :

La présente demande est également relative à une cellule tumorale, plus particulièrement myélome, comprenant au moins une protéine mutante selon l'invention, préférentiellement comprenant au moins une telle protéine mutante à sa surface, et/ou comprenant au moins un acide nucléique selon l'invention, et/ou comprenant au moins un vecteur selon l'invention, plus particulièrement un vecteur d'expression selon l'invention.

Une telle cellule tumorale, plus particulièrement un tel myélome, peut être notamment utilisée dans la production d'un hybridome (par fusion de cette cellule tumorale avec un lymphocyte B), plus particulièrement dans la production d'un hybridome producteur d'anticorps.

La présente demande est également relative à un hybridome, plus particulièrement un hybridome producteur d'anticorps, qui comprend au moins une protéine mutante selon l'invention, et/ou au moins un acide nucléique selon l'invention, et/ou au moins un vecteur selon l'invention. Un tel hybridome peut notamment être produit par mise en contact d'au moins un lymphocyte B avec au moins une cellule tumorale, plus particulièrement myélome, comprenant au moins une protéine mutante selon l'invention, préférentiellement comprenant au moins une telle protéine mutante à sa surface, et/ou comprenant au moins un acide nucléique selon l'invention, et/ou comprenant au moins un vecteur selon l'invention. Une telle cellule tumorale présente une capacité fusogénique intrinsèque : elle est donc capable de fusionner avec ledit au moins un lymphocyte B, sans mise en oeuvre de polyéthylène glycol (PEG) ou de moyens d'électroporation ou d'aucun autre des moyens qui, dans l'art antérieur, sont classiquement utilisés pour induire la fusion d'une cellule tumorale à un lymphocyte B dans le but de produire un hybridome.

### Applications biotechnologiques (cellules souches ou progénitrices) :

La présente demande est également relative à une cellule souche ou progénitrice, comprenant au moins une protéine mutante selon l'invention, préférentiellement comprenant au moins une telle protéine mutante à sa surface, et/ou comprenant au moins un acide nucléique selon l'invention, et/ou comprenant au moins un vecteur selon l'invention, plus particulièrement un vecteur d'expression selon l'invention.

Une telle cellule souche ou progénitrice présente une capacité fusogénique intrinsèque : elle est donc capable de former par fusion des syncytia.

Si cette cellule souche ou progénitrice présente en outre une capacité de différentiation en cellule musculaire, elle est alors capable de former une fibre musculaire (par fusion cellulaire et formation d'un syncytium).

La présente demande est donc également relative à une telle cellule souche ou progénitrice pour son utilisation dans la production, par exemple dans la production *in vitro*, d'une fibre musculaire.

Cette production peut par exemple être réalisée en plaçant une pluralité desdites cellules souches ou progénitrices en contact entre elles sur, ou dans, un milieu de culture permettant la prolifération de cellules souches, ou le cas échéant progénitrices, de sorte à ce que la capacité fusogène desdites cellules souches ou progénitrices puisse s'y exercer, induisant ainsi la formation d'un syncytia, plus particulièrement d'une fibre musculaire. Des exemples de milieux de culture permettant la prolifération de cellules souches, ou le cas échéant progénitrices, et qui sont en outre appropriés à l'expression de leur éventuelle capacité à se différencier en cellule musculaire, plus particulièrement en fibre musculaire, sont connus de la personne du métier, par exemple le milieu MCDB.

Des exemples de marqueurs cellulaires permettant d'observer la différentiation d'une cellule souche ou progénitrice en cellule musculaire, plus particulièrement en fibre musculaire, sont également connus de la personne du métier, par exemple CD56.

Dans la présente demande, le terme "comprenant", avec lequel "incluant" ou "contenant" est synonyme, est un terme ouvert, et n'exclut pas la présence d'un ou plusieurs élément(s), ingrédient(s) ou étape(s) de méthode additionnel(s) qui ne serait(seraient) pas explicitement indiqué(s), tandis que le terme "consistant" ou "constitué" est un terme fermé, qui exclut la présence de tout autre élément additionnel, étape, ou ingrédient qui ne serait pas explicitement exposé. Le terme "consistant essentiellement" ou " essentiellement constitué" est un terme partiellement ouvert, qui n'exclut pas la présence d'un ou plusieurs élément(s), ingrédient(s) ou étape(s) additionnel(s), dans la mesure où ce(s) élément(s), ingrédient(s) ou étape(s) additionnel(s) n'affecte(nt) pas matériellement les propriétés de base de l'invention.

Par conséquent, le terme "comprenant" (ou "comprend(comprennent)") inclut les termes "consistant", "constitué", aussi bien que les termes "consistant essentiellement" et " essentiellement constitué".

Le contenu des documents et références bibliographiques qui sont cités dans la présente demande est incorporé par référence.

Les exemples qui suivent sont donnés à titre purement illustratif, ils ne limitent l'invention en aucune façon.

### EXEMPLES

### EXEMPLE 1 : construction des mutants et mesure de leur fusogénicité

### Matériels et méthodes :

### Cellules et virus

La lignée cellulaire LLC-MK2 (lignée de cellules de rein de *Macaca mulatta*) est disponible auprès de *l'American Type Culture Collection* (ATCC) sous le numéro CCL-7. La lignée cellulaire A549 (lignée de cellules de carcinome pulmonaire humain) est disponible auprès de l'ATCC sous le numéro CCL-185.

La lignée recombinante HuH7-Tat (lignée de cellules d'hépatome humain) est disponible par transduction de cellules de la lignée HuH-7 par HIV-1 Tat.

La lignée HuH-7 est disponible auprès de la Collection Japonaise des Bioressources de Recherches *-Japanese Collection of Research Bioresources-* sous la référence JCRB0403. La transduction de la lignée HuH-7 par HIV-Tat a été réalisée à l'aide du vecteur rétroviral LXSN-tat transduisant le plasmide Tat.

Les cellules LLC-MK2, A549 et HuH7-Tat ont été cultivées en milieu EMEM (*Eagle's Minimum Essential Medium*) ou DMEM (*Dulbecco*/*Vogt Modified Eagles' essential minimal Medium*) avec 5% de sérum de veau foetal.

La souche PIV-5 WR a été obtenue auprès de l'ATCC (numéro ATCC VR-288), et a été cultivée sur des cellules LLC-MK2 comme décrit dans Terrier *et al.* 2008.

### Extraction d'ARN, RT-PCR et clonage

L'ARN viral a été extrait du surnageant obtenu à partir d'une infection de cellules LLC-MK2 par PIV-5, à l'aide du kit *Absolutely RNA*® *microprep kit* (Stratagene, USA), en suivant les instructions données par le fournisseur. La transcription inverse a été réalisée à l'aide d'hexamères aléatoires pd(N)6 (Amersham Biosciences, GB) et d'une transcriptase inverse (*Reverse Transcriptase* ; RT) du virus de la myéloblastose aviaire (*Avian Myeloblastosis Virus ;* AMV) (transcriptase inverse AMV-RT disponible auprès de Promega).

L'amplification de la séquence complète de F PIV-5 a été réalisée avec un couple d'amorces conçu à partir de la séquence nucléotidique de PIV-5 disponible dans les bases de données (numéro d'accès GenBank AB021962).

Le couple d'amorces utilisé était le suivant :
Amorce sens (SEQ ID NO: 1) :
   5' TTGCGGCCGCATGGGTACTATAA 3'
Amorce antisens (SEQ ID NO: 2) :
   5' CCGCTCGAGTTATGATAAACAAAATTCTCC 3'

L'amplification a été réalisée selon le protocole suivant : 95°C pendant 2 min, puis 39 cycles (95°C/30 s 55°C/1 min 72°C/3 min) et une élongation finale de 10 min à 72°C. L'ADN complémentaire de F PIV-5 a été cloné dans le plasmide d'expression pcDNA3.1(+) au niveau des sites *NotI* et *XhoI* au niveau du site multiple de clonage (*cf.* Figure 4).

Les produits PCR et les plasmides ont été purifiés, respectivement par les kits *Nucleospin*® *ExtractII* et *Nucleospin*® *plasmid* (Macherey Nagel, Germany), en suivant les instructions indiquées par le fournisseur.

L'ensemble des séquençages réalisés dans cette étude a été réalisé par MWG Biotech (Ebersberg, Germany).

### Mutagenèse dirigée par amplification en chaîne par polymérase (PCR)

Les protéines mutantes de protéine F de PIV-5 ont été réalisées par mutation dirigée au sein du plasmide pcDNA3.1 codant la protéine de fusion F PIV-5. La(les) mutation(s) a(ont) été générées par PCR à l'aide d'amorces complémentaires, en suivant le protocole indiqué par le fournisseur (*QuickChange*® *Site-Directed Mutagenesis system* disponible auprès de Stratagene). La liste des amorces utilisées est donnée dans le tableau 2 ci-dessous. L'ensemble des plasmides a été contrôlé par séquençage.

**Tableau 2 : liste des amorces**

| **Mutation** | **Région ciblée dans PIV-5** | | **Séquences des amorces utilisées pour la mutagénèse par PCR** | **SEQ ID NO:** |
|---|---|---|---|---|
| **L22P** | F2 | Sens | 5' GGAGCAGGCAGCCTTGATC**C**AGC**T**GCTCTCATGCAAATCGG 3' | 3 |
| | | Antisens | 5' CCGATTTGCATGAGAGC**A**GCT**G**GATCAAGGCTGCCTGCTCC 3' | 4 |
| **K132E** | HR1 | - | Mutation pré-existante | - |
| **V290A** | HR3 | - | Mutation pré-existante | - |
| **I49A** | F2 | Sens | 5' GGCCTCATCAGCATTC**GC**TGTTGTGAAGTTAATGCC 3' | 5 |
| | | Antisens | 5' GGCATTAACTTCACAACA**GC**GAATGCTGATGAGGCC 3' | 6 |
| **V402A** | entre HR3 et HR2 | Sens | 5' CAGCCAAGTTCATCTCCTG**C**AACTGTCATTGACATGTAC 3' | 7 |
| | | Antisens | 5'GTACATGTCAATGACAGTT**G**CAGGAGAGTGAACTTGGCTG3' | 8 |
| **S443P** | amont de HR2 | Sens | 5' GCTTGAATCATCTCAGATCTTG**T**CCATTGATCCGTTGGATATATCCC 3' | 9 |
| | | Antisens | 5' GGGATATATCCAACGGATCAATGG**A**CAAGATCTGAGATGATTCAAGC 3' | 10 |
| **L447P** | amont de HR2 | Sens | 5' CTCAGATCTTGTCCATTGATCCG**CC**GGATATATCCCAGAATCTAGCTGCG 3' | 11 |
| | | Antisens | 5' CGCAGCTAGATTCTGGGATATATCC**GG**CGGATCAATGGACAAGATCTGAG 3' | 12 |
| **I449P** | amont de HR2 | Sens | 5' CTTGTCCATTGATCCGTTGGAT**CC**ATCCCAGAATCTAGCTGCGGTG 3' | 13 |
| | | Antisens | 5'CACCGCAGCTAGATTCGTTGGATTTCTCCCAGAATCTAGCTGCGG3' | 14 |
| **I449F** | amont de HR2 | Sens | 5' GCCCATTGATCCGTTGGAT**T**T**C**TCCCAGAATCTAGCTGCGG 3' | 15 |
| | | Antisens | 5'CCGCAGCTAGATTCTGGGA**G**A**A**ATCCAACGGATCAATGGGC 3' | 16 |
| **T147V** | HR1 | Sens | 5'CTCAAAAATGCAATCCAAAAA**GT**AAATGCAGCAGTTGCAGATG3' | 17 |
| | | Antisens | 5' CATCTGCAACTGCTGCATTT**AC**TTTTTGGATTGCATTTTTGAG 3' | 18 |
| **T158V** | HR1 | Sens | 5' GCAGATGTGGTCCAGGCC**GT**ACAATCACTAGGAACGGC 3' | 19 |
| | | Antisens | 5' GCCGTTCCTAGTGATTGT**AC**GGCCTGGACCACATCTGC 3' | 20 |
| **A463V** | HR2 | Sens | 5' GTGAATAAGAGTCTAAGTGATG**T**ACTACAACACTTAGCACAAAGTG 3' | 21 |
| | | Antisens | 5' CACTTTGTGCTAAGTGTTGTAGT**A**CATCACTTAGACTCTTATTCAC 3' | 22 |

La mutation 443P est théoriquement pré-existante dans la protéine F de l'isolat WR. Toutefois, dans l'échantillon de cet isolat que les inventeurs ont reçu de l'ATCC, cette mutation n'était en fait pas présente. Elle a donc dû être introduite par les inventeurs.

### Transfection des cellules

Les cellules ont été transfectées par les plasmides à l'aide du réactif ExGen500 (Fermentas), en suivant les instructions indiquées par le fournisseur. Un à trois microgrammes d'ADN plasmidique ont été ajoutés sur les cellules (à 70 à 80% confluence) pour 48h. L'efficacité de la transfection a été estimée à l'aide d'un plasmide codant la protéine vert fluorescent (*Green Fluorescence Protein* ; GFP).

### Immunofluorescence par microscopie confocale

Les cellules transfectés ont été fixées à l'aide de paraformaldéhyde (1% v/v) en tampon phosphate (*Phosphate Buffer Saline ;* PBS) puis lavées deux fois. Les tapis cellulaires ont été incubés en présence d'un anticorps monoclonal dirigé contre la protéine de fusion F PIV-5, en l'occurrence l'anticorps monoclonal F1a décrit dans Randall *et al.* 1987, dilué au 1/10 en PBS pendant 3h. L'anticorps monoclonal F1a a été obtenu par immunisation de souris contre un isolat de PIV-5 (en l'occurrence l'isolat LN), préparation des hybridomes et sélection des anticorps anti-F spécifiques.

Les tapis cellulaires ont ensuite été lavés et incubés avec un anticorps secondaire anti-souris IgG-Alexa Fluor® 633 (Invitrogen) dilué au 1/200 en PBS pendant 30 minutes. Après rinçages, les cellules ont été incubées pendant 10 minutes avec du Dapi (4',6' Di Amidino-2-Phényl Indole) au 1/1000 mélangé ou non avec de l'agglutinine de germe de blé (*Wheat Germ Agglutinin* ; WGA) couplée à de l'Alexa Fluor® 488 (WGA- Alexa Fluor® disponible auprès de Invitrogen) au 1/200 en tampon phosphate (*Phosphate Buffer Saline* ; PBS). Les images ont été acquises à l'aide d'un microscope confocal TCS SP2 (Leica).

### Cytométrie en flux

La cytométrie en flux a été réalisée comme précédemment décrit dans la littérature (Horvat et Lamb 1992). Des cellules A549 ont été transfectées par les plasmides codant les différents Fus et ont été déposées sur glace. Les tapis cellulaires ont été rincés par du tampon phosphate PBS (*Phosphate Buffer Saline ;* PBS) comportant 1% d'azide de sodium. Un anticorps monoclonal anti-protéine F de PIV-5 (en l'occurrence l'anticorps monoclonal F1a) a ensuite été ajouté sur les tapis (1/500 en tampon phosphate PBS à 1% en sérum de veau foetal), et incubé 30 minutes à 4°C. Les tapis ont été ensuite rincés et incubés en présence d'un anticorps secondaire anti-souris couplé à l'Alexa Fluor® 488 au 1/1000 (Invitrogen). Après rinçages, les cellules ont été décollées doucement à l'aide de 500µL de tampon phosphate PBS à 0,5mM en EDTA (acide éthylène-diamine-tétraacétique). Les cellules ont été transférées dans des tubes dédiés à la cytométrie en flux contenant 500µL d'une solution de paraformaldéhyde 1%. L'intensité de fluorescence de 5000 cellules a été mesurée sur un cytomètre en flux trieur de cellules (*Fluorescence-activated cell sorting* ; FACS), en l'occurrence sur le *FACSVantage*™ *SE flow* de Becton Dickinson.

### Test de fusion semi-quantitatif (scores de fusion établis en fonction de la taille du syncytium et du nombre de noyaux)

Les tapis transfectés par les différents plasmides d'expression Fus et observés en immunofluorescence ont permis une analyse semi-quantitative. Cette analyse a consisté à déterminer un score de fusion pour chacun des mutants suivant les critères suivants :
- La fusion ou non (simples agglomérats), notée -/+ ;
- La taille du syncytium, notée sur une échelle de 1 à 5 ;
- Le nombre de noyaux, noté sur une échelle de 1 à 5.

Le score ainsi calculé est l'addition des deux notes ; la note maximale théorique est donc de 10 et correspond à un syncytium de taille maximale avec un nombre maximum de noyaux.

### Test de fusion quantitatif (mesure de l'activité luciférase)

Afin de quantifier la fusion cellule-cellule, des cellules A549 **«** donneuses » (2,5 millions de cellules par puits de plaque à 6 puits) ont été co-transfectées avec 2µg de plasmide pcDNA3.1 codant les différentes protéines mutantes *Fus* ainsi que 50ng d'un plasmide exprimant la luciférase sous la dépendance de la longue répétition terminale du VIH-1 (*Long Terminal Repeat* ; LTR) (Lavillette *et al.* 2007).

A titre de témoin négatif, des cellules ont été co-transfectées avec 2µg de plasmide pcDNA3.1 vide.

Douze heures après la transfection, les cellules « donneuses » ont été détachées à l'aide de tampon phosphate (PBS) à 0,5 mM en EDTA, et ont été comptées puis replacées dans de nouvelles plaques 6 puits (10⁵ cellules/puits). Des cellules « indicatrices » HuH7-Tat (4.10⁵ cellules/puits) ont été décollées à l'aide du tampon PBS-EDTA, puis rincées et ajoutées aux cellules « donneuses ».

L'activité luciférase a été mesurée à 72h de co-culture à l'aide d'un kit de mesure de l'activité luciférase, en l'occurrence le *Luciferase Assay system (E1500)* kit de Promega, en suivant les indications données par le fournisseur.

### Résultats :

Des protéines mutantes construites et produites par les inventeurs sont présentées en tableau 3 ci-dessus.

Dans ce tableau 3, les inventeurs ont organisé les différentes mutations selon la fonction qu'ils leur ont attribuée, à savoir :
- implication dans la fonction d'autonomie vis-à-vis de HN : positions 22, 132 et 290 de la protéine F de PIV-5 ;
- implication dans la fonction de pré-fusion : positions 49, 402, 443, 447 et 449 de la protéine F de PIV-5 ;
- implication dans la fonction de post-fusion: positions 147, 158 et 463 de la protéine F de PIV-5.

En figures 5A, 5B, 5C et 5D, sont illustrées les positions des mutations du tableau 3.

Des protéines mutantes ont ainsi été construites, produites et testées par les inventeurs.

**Tableau 3 : liste de protéines réalisées**

| | **Autonomie** | | | **Pré-fusion** | | | | | | **Post-fusion** | | |
|---|---|---|---|---|---|---|---|---|---|---|---|---|
| | **L22P** | **K132E** | **V290A** | **I49A** | **V402A** | **S443P** | **L447P** | **I449P** | **I449F** | **T147V** | **T158V** | **A463V** |
| Fus1 (SEQ ID NO: 47) | | + | + | | | | | | | | | |
| Fus1.1 (SEQ ID NO: 48) | | + | + | + | | | | | | | | |
| Fus 1.2 (SEQ ID NO: 49) | | + | + | | + | | | | | | | |
| Fus2 (SEQ ID NO: 50) | | + | + | | | + | | | | | | |
| Fus3 (SEQ ID NO: 51) | + | + | + | | | | | | | | | |
| Fus3.1 (SEQ ID NO: 52) | + | + | + | | | | | | | + | | |
| Fus3.2 (SEQ ID NO: 53) | + | + | + | | | | | | | + | + | |
| Fus 3.3 (SEQ ID NO: 54) | + | + | + | | | | | | | | + | |
| Fus4 (SEQ ID NO: 55) | + | + | + | + | | | | | | | | |
| Fus5 (SEQ ID NO: 56) | + | + | + | | + | | | | | | | |
| Fus6 (SEQ ID NO: 57) | + | + | + | | | + | | | | | | |
| Fus 6.1 (SEQ ID NO: 58) | + | + | + | | | + | | | | + | | |
| Fus 6.2 (SEQ ID NO: 59) | + | + | + | | | + | | | | | + | |
| Fus 6.3 (SEQ D NO: 60) | + | + | + | | | + | | | | + | + | |
| Fus7 (SEQ ID NO: 61) | + | + | + | | | | + | | | | | |
| Fus 7.1 (SEQ ID NO: 62) | + | + | + | | | | + | | | + | | |
| Fus 7.2 (SEQ ID NO: 63) | + | + | + | | | | + | | | | + | |
| Fus 7.3 (SEQ ID NO: 64) | + | + | + | | | | + | | | + | + | |
| Fus8 (SEQ ID NO: 65) | + | + | + | | | | | + | | | | |
| Fus9 (SEQ ID NO: 66) | + | + | + | | | | | | + | | | |
| Fus 10 (SEQ ID NO: 67) | + | + | + | + | | | | + | | | | |
| Fus 10.4 (SEQ ID NO: 68) | + | + | + | | + | | | + | | + | + | + |
| Fus 10.5 (SEQ ID NO: 69) | + | + | + | + | + | | | + | | + | + | + |
| Fus11 (SEQ ID NO: 70) | + | + | + | | + | | | + | | | | |
| Fus8.1 (SEQ ID NO: 71) | + | + | + | | | | | + | | + | | |
| Fu8.2 (SEQ ID NO: 72) | + | + | + | | | | | + | | | + | |
| Fus8.4 (SEQ ID NO: 73) | + | + | + | | | | | + | | + | + | |
| Fus8.5 (SEQ ID NO: 74) | + | + | + | | | | | + | | + | | + |
| Fus8.6 (SEQ ID NO: 75) | + | + | + | | | | | + | | | + | + |
| Fus8.7 (SEQ ID NO: 76) | + | + | + | | | | | + | | + | + | + |
| Fus10.1 (SEQ ID NO: 77) | + | + | + | + | | | | + | | | + | |
| Fus10.2 (SEQ ID NO: 78) | + | + | + | + | | | | + | | + | + | |
| Fus10.3 (SEQ ID NO: 79) | + | + | + | + | | | | + | | + | + | + |

La séquence de protéine F de PIV-5 qui a été mise en oeuvre lors de la construction et la production de ces protéines mutantes était une séquence alternative de protéine F d'isolat WR. Cette séquence alternative était identique à la séquence de SEQ ID NO: 31 (séquence Genbank), à l'exception de l'acide aminé en position 443 qui était S et non P (séquence alternative « SEQ ID NO: 31 avec S en 443 »).

Les séquences de SEQ ID NO: 47 à 79 sont donc les séquences qui résultent du remplacement, au sein de ladite séquence alternative « SEQ ID NO: 31 avec S en 443 », des acides aminés indiqués pour chacune de ces séquences dans le tableau 3 ci-dessus.

Une illustration des observations qui ont été faites au microscope lors des tests de fusion semi-quantitatifs est présentée en Figure 6A.

Les scores obtenus à l'issue des tests de fusion semi-quantitatifs sont présentés en Figure 6B.

Les protéines mutantes Fus 6, Fus 6.1, Fus 6.2 et Fus 6.3 ont conduit à l'agglutination de nombreuses cellules, mais à aucune fusion cellulaire.

Les protéines mutantes Fus 3.3, Fus 3.1, Fus2, Fus 1.1, Fus 1.2 et Fus 1 donnent un score de fusion nul.

Les protéines mutantes Fus9, Fus7, Fus 3, Fus5 et Fus4 donnent de faibles scores de fusion.

Au-delà du score de fusion de Fus4, se détachent un ensemble de protéines mutantes à score de fusion significatif, à savoir :
- le groupe des protéines mutantes qui ont en commun de comprendre les trois mutations d'autonomie et la mutation de pré-fusion 449P, telles que les protéines mutantes Fus8, Fus10, Fus10.4, Fus10.5, Fus11, Fus8.1, Fus8.2, Fus8.4, Fus8.5, Fus8.6, Fus8.7, Fus10.1, Fus10.2, Fus10.3, et
- le groupe des protéines mutantes qui ont en commun de comprendre les trois mutations d'autonomie, la mutation de pré-fusion 447P et au moins une mutation de post-fusion (147V ou 158V), telles que Fus7.1, Fus7.2 et Fus7.3.

Les Figures 7A et 7B présentent une illustration des observations faites au microscope et présentent les scores de fusion pour une sélection des protéines mutantes testées, à savoir le groupe des protéines mutantes qui ont en commun de comprendre les trois mutations d'autonomie et la mutation de pré-fusion 449P, telles que les protéines mutantes Fus8, Fus10, Fus10.4, Fus10.5, Fus11, Fus8.1, Fus8.2, Fus8.4, Fus8.5, Fus8.6, Fus8.7, Fus10.1, Fus10.2, Fus10.3.

Les Figures 8A, 8B et 8C donnent une illustration des résultats des résultats obtenus par le test de fusion dit quantitatif (test par luciférase).

Les Figures 8A et 8C montrent que les protéines mutantes qui ont en commun de comprendre les trois mutations d'autonomie et la mutation de pré-fusion 449P, telles que la protéine mutante Fus8, présentent une capacité fusogène plus forte (en fait, plus de trois fois plus forte) que celle de la protéine Fus3, même lorsque Fus3 est associée à la glycoprotéine HN, à titre de mimétique de la situation naturelle (Figure 8A).

La protéine mutante Fus8.7 présente une capacité fusogène plus forte que la protéine mutante Fus8 (presque deux fois plus importante), elle même plus forte que la protéine Fus3 (plus de six fois plus importante) même lorsque Fus3 est associé à la glycoprotéine HN, à titre de mimétique de la situation naturelle (Figure 8C).

La Figure 8B illustre le fait que la capacité fusogénique du groupe des protéines mutantes qui ont en commun de comprendre les trois mutations d'autonomie et la mutation de pré-fusion 449P, n'est pas due à une simple corrélation avec le niveau d'expression surfacique obtenu. En effet, on peut notamment constater que l'expression de Fus8 est environ moitié moindre que celle de Fus3 (Figure 8B).

### EXEMPLE 2: Substitution du site de clivage naturel par le site d'une enzyme exprimée spécifiquement par le tissu tumoral métastatique

Les protéines mutantes de l'invention, et plus particulièrement celles décrites en exemple 1 ci-dessus, ont été plus avant modifiées par substitution du site de clivage naturel de la protéine F native, par exemple pour le remplacer par un site de clivage tissu-spécifique.

A titre illustratif, sont ici présentées des protéines mutantes de l'invention dont le site de clivage naturel a été substitué par le site d'une enzyme exprimée spécifiquement par le tissu tumoral métastatique, à savoir la métallo-protéase matricielle 9 (MMP-9).

La Figure 9 illustre la substitution ainsi opérée pour des protéines mutantes de la protéine F de PIV-5.

Le site de clivage naturel de la protéine F de PIV-5 est :
**RRRRR** (SEQ ID NO: 23).

Un exemple de fragment de séquence de protéine F comprenant le site de clivage naturel de la protéine F de PIV-5 est :
IGENLETIRNQLIPT**RRRRR**FAGVVIGL (SEQ ID NO: 24).

La séquence consensus d'un site de clivage MMP-9 est :
**PXXHyS/T** (SEQ ID NO: 27)
avec X = n'importe quel acide aminé, et
avec Hy = n'importe quel acide aminé hydrophobe (c'est-à-dire n'importe quel acide aminé parmi F, M, V, L, I).

Un exemple de site de clivage MMP-9 est :
**PRRIT** (SEQ ID NO: 28).

Un exemple de fragment de séquence de protéine F mutante de l'invention comprenant un site de clivage MMP-9 est :
IGENLETIRNQLIPT**PRRIT**FAGVVIGL (SEQ ID NO: 29).

### Matériels et Méthodes :

Les protéines mutantes de la protéine F de PIV-5 ont été produites comme décrit en exemple 1 ci-dessus.

Le remplacement du site de clivage naturel par le site de clivage sélectionné, en l'occurrence, le site de clivage MMP-9 de SEQ ID NO : 28, a été réalisé comme suit :
Le remplacement du site de clivage a été réalisé par 3 mutagenèses dirigées successives au sein du plasmide pcDNA3.1 codant la protéine de fusion F PIV-5. Les mutations ont été générées par PCR à l'aide d'amorces complémentaires, en suivant le protocole indiqué par le fournisseur (*QuickChange*® *Site*-*Directed Mutagenesis system* disponible auprès de Stratagene). L'ensemble des plasmides a été contrôlé par séquençage.

### 1^{ÈRE} MUTATION R98P

SENS 5' CCAGTTGATTCCAACTCCGAGGAGACGCCGGTTTGC 3' (SEQ ID NO: 80)
ANTISENS 5' GCAAACCGGCGTCTCCTCGGAGTTGGAATGAACTGG 3' (SEQ ID NO: 81)

### 2^{NDE} MUTATION R1011

SENS 5' GATTCCAACTCCGAGGAGAATCCGGTTTGCAGGAGTGGTG 3' (SEQ ID NO: 82)
ANTISENS 5'CACCACTCCTGCAAACCGGATTCTCCTCGGAGTTGGAATC 3' (SEQ ID NO: 83)

### 3^{ÈME} MUTATION R102T

SENS 5' GATTCCAACTCCGAGGAGAATCACGTTTGCAGGAGTGGTGATTGG 3' (SEQ ID NO: 84)
ANTISENS 5' CCAATCACCACTCCTGCAAACGTGATTCTCCTCGGAGTTGGAATC 3' (SEQ ID NO: 85)

### Transfection des cellules

Les cellules ont été transfectées par les plasmides à l'aide du réactif ExGen500 (Fermentas), en suivant les instructions indiquées par le fournisseur. Un à trois microgrammes d'ADN plasmidique ont été ajoutés sur les cellules (à 70 à 80% confluence) pour 48h. L'efficacité de la transfection a été estimée à l'aide d'un plasmide codant la protéine vert fluorescent (*Green Fluorescence Protein ;* GFP).

### Immunofluorescence par microscopie confocale

Les cellules transfectés ont été fixées à l'aide de paraformaldéhyde (1% v/v) en tampon phosphate (*Phosphate Buffer Saline* ; PBS) puis lavées deux fois. Les tapis cellulaires ont été incubés en présence d'un anticorps monoclonal dirigé contre la protéine de fusion F PIV-5, en l'occurrence l'anticorps monoclonal F1a décrit dans Randall *et al.* 1987, dilué au 1/10 en PBS pendant 3h. L'anticorps monoclonal F1a a été obtenu par immunisation de souris contre un isolat de PIV-5 (en l'occurrence l'isolat LN), préparation des hybridomes et sélection des anticorps anti-F spécifiques.

Les tapis cellulaires ont ensuite été lavés et incubés avec un anticorps secondaire anti-souris IgG-Alexa Fluor® 633 (Invitrogen) dilué au 1/200 en PBS pendant 30 minutes. Après rinçages, les cellules ont été incubées pendant 10 minutes avec du Dapi (4',6' Di Amidino-2-Phényl Indole) au 1/1000 mélangé ou non avec de l'agglutinine de germe de blé (*Wheat Germ Agglutinin* ; WGA) couplée à de l'Alexa Fluor® 488 (WGA- Alexa Fluor® disponible auprès de Invitrogen) au 1/200 en tampon phosphate (*Phosphate Buffer Saline* ; PBS). Les images ont été acquises à l'aide d'un microscope confocal TCS SP2 (Leica).

### Résultats :

Les inventeurs ont ainsi obtenu des protéines mutantes telles que les protéines Fus8M et Fus8.7M (SEQ ID NO: 88 et 89, respectivement) qui dérivent des protéines mutantes Fus8 et Fus8.7 par remplacement du site de clivage naturel par le site MMP-9 de SEQ ID NO: 28.
Fus8 (SEQ ID NO: 65) :

MGTIIQFLVV SCLLAGAGSL DPAALMQIGV IPTNVRQLMY YTEASSAFIV VKLMPTIDSP
ISGCNITSIS SYNATVTKLL QPIGENLETI RNQLIPTRRR RRFAGVVIGL AALGVATAAQ
VTAAVALVKA NENAAAILNL KNAIQKTNAA VADVVQATQS LGTAVQAVQD HINSVVSPAI
TAANCKAQD AIIGSILNLYL TELTTIFHNQ ITNPALSPIT IQALRILLGS TLPTVVEKSF

NTQISAAELL SSGLLTGQIV GLDLTYMQMV IKIELPTLTV QPATQIIDLA TISAFINNQE
VMAQLPTRVM VTGSLIQAYP ASQCTITPNT VYCRYNDAQV LSDDTMACLQ GNLTRCTFSP
VVGSFLTRFV LFDGIVYANC RSMLCKCMQP AAVILQPSSS PVTVIDMYKC VSLQLDNLRF
TITQLANVTY NSTIKLESSQ ILSIDPLDPS QNLAAVNKSL SDALQHLAQS DTYLSAITSA
TTTSVLSIIA ICLGSLGLIL IILLSVVVWK LLTIVAANRN RMENFVYHK

Le site de clivage naturel est en positions 98 à 102 (RRRRR).
Fus8M (SEQ ID NO: 88) :

MGTIIQFLVV SCLLAGAGSL DPAALMQIGV IPTNVRQLMY YTEASSAFIV VKLMPTIDSP
ISGCNITSIS SYNATVTKLL QPIGENLETI RNQLIPTPRR ITFAGVVIGL AALGVATAAQ
VTAAVALVKA NENAAAILNL KNAIQKTNAA VADVVQATQS LGTAVQAVQD HINSVVSPAI
TAANCKAQD AIIGSILNLYL TELTTIFHNQ ITNPALSPIT IQALRILLGS TLPTVVEKSF
NTQISAAELL SSGLLTGQIV GLDLTYMQMV IKIELPTLTV QPATQIIDLA TISAFINNQE
VMAQLPTRVM VTGSLIQAYP ASQCTITPNT VYCRYNDAQV LSDDTMACLQ GNLTRCTFSP
VVGSFLTRFV LFDGIVYANC RSMLCKCMQP AAVILQPSSS PVTVIDMYKC VSLQLDNLRF
TITQLANVTY NSTIKLESSQ ILSIDPLDPS QNLAAVNKSL SDALQHLAQS DTYLSAITSA
TTTSVLSIIA ICLGSLGLIL IILLSVVVWK LLTIVAANRN RMENFVYHK

Le site de clivage naturel RRRRR a été remplacé par le site PRRIT (site de MMP-9).
Fus8.7 (SEQ ID NO: 76) :

MGTIIQFLVV SCLLAGAGSL DPAALMQIGV IPTNVRQLMY YTEASSAFIV VKLMPTIDSP
ISGCNITSIS SYNATVTKLL QPIGENLETI RNQLIPTRRR RRFAGVVIGL AALGVATAAQ
VTAAVALVKA NENAAAILNL KNAIQKVNAA VADVVQAVQS LGTAVQAVQD HINSVVSPAI
TAANCKAQD AIIGSILNLYL TELTTIFHNQ ITNPALSPIT IQALRILLGS TLPTVVEKSF
NTQISAAELL SSGLLTGQIV GLDLTYMQMV IKIELPTLTV QPATQIIDLA TISAFINNQE
VMAQLPTRVM VTGSLIQAYP ASQCTITPNT VYCRYNDAQV LSDDTMACLQ GNLTRCTFSP
VVGSFLTRFV LFDGIVYANC RSMLCKCMQP AAVILQPSSS PVTVIDMYKC VSLQLDNLRF
TITQLANVTY NSTIKLESSQ ILSIDPLDPS QNLAAVNKSL SDVLQHLAQS DTYLSAITSA
TTTSVLSIIA ICLGSLGLIL IILLSVVVWK LLTIVAANRN RMENFVYHK

Le site de clivage naturel est en positions 98 à 102 (RRRRR).
Fus8.7M (SEQ ID NO: 89) :

MGTIIQFLVV SCLLAGAGSL DPAALMQIGV IPTNVRQLMY YTEASSAFIV VKLMPTIDSP
ISGCNITSIS SYNATVTKLL QPIGENLETI RNQLIPTPRR ITFAGVVIGL AALGVATAAQ
VTAAVALVKA NENAAAILNL KNAIQKVNAA VADVVQAVQS LGTAVQAVQD HINSVVSPAI
TAANCKAQD AIIGSILNLYL TELTTIFHNQ ITNPALSPIT IQALRILLGS TLPTVVEKSF
NTQISAAELL SSGLLTGQIV GLDLTYMQMV IKIELPTLTV QPATQIIDLA TISAFINNQE
VMAQLPTRVM VTGSLIQAYP ASQCTITPNT VYCRYNDAQV LSDDTMACLQ GNLTRCTFSP
VVGSFLTRFV LFDGIVYANC RSMLCKCMQP AAVILQPSSS PVTVIDMYKC VSLQLDNLRF
TITQLANVTY NSTIKLESSQ ILSIDPLDPS QNLAAVNKSL SDVLQHLAQS DTYLSAITSA
TTTSVLSIIA ICLGSLGLIL IILLSVVVWK LLTIVAANRN RMENFVYHK

Le site de clivage naturel RRRRR a été remplacé par le site PRRIT (site de MMP-9).

Les protéines mutantes obtenues sont aussi fonctionnelles que les protéines mutantes à site de clivage naturel dont elles dérivent (induction de syncytia larges comprenant de nombreux noyaux, expression de surface et quantification d'un haut niveau de fusogénicité).

Ce résultat est illustré par la figure 9 (immunofluorescence par microscopie confocale réalisée comme décrit en exemple 1).

La modification du site de clivage ne semble pas modifier de manière significative la capacité fusogène (scores de fusion très proches) et la fonctionnalité des protéines mutantes de l'invention.

Pour ce qui concerne les protéines mutantes de l'invention qui dérivent de la protéine F de PIV-2, la même opération d'introduction ou substitution de site de clivage peut être réalisée.

Le site de clivage naturel de la protéine F de PIV-2 est KTRQKR (SEQ ID NO: 25), un exemple de fragment de séquence de protéine F comprenant le site de clivage naturel de la protéine F de PIV-2 étant LTPLIENLSKISTVTDT**KTRQKR**FAGVVVGLAALGVA (SEQ ID NO: 26). Dans les protéines mutantes de l'invention, un site de clivage autre que ce site naturel de clivage peut être introduit ou venir en substitution, par exemple un site de clivage tissu-spécifique, plus particulièrement un site de clivage d'une enzyme exprimée spécifiquement par le tissu tumoral métastatique, telle que la métallo-protéase matricielle 9 (MMP-9 ; site de SEQ ID NO: 27 ou 28 par exemple).

### Références bibliographiques

Baker et al. 1999, Mol Cell. 3(3):309-19.
Chatziandreou et al. 2004, Journal of General Virology 85 : 3007-3016.
Horvat et Lamb 1992, J. Virol. 66(4) : 2443-2455.
Ito et al. 1997, J Virol. 71(12) : 9855-9858.
Ito et al. 2000, J Gen Virol. 81(Pt 3):719-727.
Gardner et Dutch 2007, J. Virol. 81(15):8303-14.
Gardner et al. 2007, Biochemistry 46(17):5094-5105.
Lavillette D. et al. 2007, J. Virol. 81(16) : 8752-8765
Paterson et al. 2000, Virology 270(1):17-30.
Randall et al. 1987, J. Gen. Virol. 68(Pt11) : 2769-2780
Russell et al. 2003, J. Cell Biol. 163(2):363-74.
Terrier et al. 2008, Journal of Clinical Virology, 2008, 43(1): 86-92.
West et al. 2005, J Virol. 79(3):1543-1551.

## Revendications

1. Protéine mutante, dont la séquence en acides aminés comprend une séquence qui diffère de celle de la protéine F d'un virus PIV-5 ou PIV-2 :
- par remplacement :
- de l'acide aminé qui, dans la séquence de ladite protéine F de virus PIV-5, est en position 22, ou qui, dans la séquence de ladite protéine F de virus PIV-2, est en position 24, par l'acide aminé P, et
- de l'acide aminé qui, dans la séquence de ladite protéine F de virus PIV-5, est en position 132, ou qui, dans la séquence de ladite protéine F de virus PIV-2, est en position 133, par l'acide aminé E, et
- de l'acide aminé qui, dans la séquence de ladite protéine F de virus PIV-5, est en position 290, ou qui, dans la séquence de ladite protéine F de virus PIV-2, est en position 294, par l'acide aminé A, et
- de l'acide aminé qui, dans la séquence de ladite protéine F de virus PIV-5, est en position 449, ou qui, dans la séquence de ladite protéine F de virus PIV-2, est en position 439, par l'acide aminé P,
- et, optionnellement :
- par substitution du site de clivage natif de ladite protéine F par un autre site de clivage enzymatique, et/ou par insertion dans ladite protéine F d'un site de clivage enzymatique autre que le site de clivage natif de cette protéine F, et/ou
- par délétion d'une partie C-terminale de ladite protéine F, ladite partie C-terminale s'étendant en direction N-terminale à partir du dernier acide aminé à l'extrémité C-terminale de la protéine, mais sans s'étendre au-delà du domaine HR2 de ladite protéine F,
la séquence de ladite protéine F de virus PIV-5 ou PIV-2 comprenant la séquence de SEQ ID NO: 34.

2. La protéine mutante selon la revendication 1, **caractérisée en ce que** la séquence de ladite protéine F est une protéine F de PIV-5.

3. La protéine mutante selon la revendication 2, **caractérisée en ce que** la séquence de ladite protéine F consiste en :
- la séquence de SEQ ID NO: 31, ou la séquence alternative qui est identique à la séquence de SEQ ID NO: 31 à l'exception de l'acide aminé en position 443, qui est S dans cette séquence alternative (au lieu de P en SEQ ID NO: 31), ou en
- une séquence variante de cette séquence de SEQ ID NO: 31 ou de ladite séquence alternative, cette séquence variante :
o étant d'une taille identique à celle de SEQ ID NO: 31, ou étant d'une taille supérieure d'un maximum de 7 acides aminés à celle de SEQ ID NO: 31, ou étant d'une taille inférieure d'un maximum de 7 acides aminés à celle de SEQ ID NO: 31, et
o présentant une identité de séquence de plus de 95%, préférentiellement d'au moins 96%, plus préférentiellement d'au moins 97%, par rapport à la séquence de SEQ ID NO: 31 ou à ladite séquence alternative, cette identité étant calculée sur la longueur de la séquence de SEQ ID NO: 31 ou, le cas échéant, de ladite séquence alternative.

4. La protéine mutante selon la revendication 3, **caractérisée en ce que** la séquence de ladite protéine F consiste en la séquence de SEQ ID NO: 31 modifiée par les remplacements d'acides aminés indiqués dans le tableau 1 en colonne « W3A » ou « MIL » ou « DEN » ou « LN » ou « MEL » ou « Cryptovirus » ou « CPI+ » ou « CPI- » ou « H221 » ou « 78524 » ou « T1 » ou « SER ».

5. La protéine mutante selon l'une quelconque des revendications 2 à 4, **caractérisée en ce que** sa séquence en acides aminés ne comprend pas d'autre mutation par rapport à ladite séquence de protéine F de PIV-5.

6. La protéine mutante selon la revendication 5, **caractérisée en ce que** ladite séquence qui diffère de celle de ladite protéine F est la séquence de SEQ ID NO: 65.

7. La protéine mutante selon l'une quelconque des revendications 2 à 4, **caractérisée en ce que** ladite séquence qui diffère de celle de ladite protéine F en diffère en outre par :
- au moins une mutation de pré-fusion choisie parmi :
- le remplacement de l'acide aminé en position 49 par l'acide aminé A,
- le remplacement de l'acide aminé en position 402 par l'acide aminé A,
- le remplacement de l'acide aminé en position 443 par l'acide aminé P,
- le remplacement de l'acide aminé en position 447 par l'acide aminé P,
et/ou par
- au moins une mutation de post-fusion choisie parmi :
- le remplacement de l'acide aminé en position 147 par un acide aminé hydrophobe,
- le remplacement de l'acide aminé en position 158 par un acide aminé hydrophobe.

8. La protéine mutante selon l'une quelconque des revendications 2 à 4 et 7, **caractérisée en ce que** ladite séquence qui diffère de celle de ladite protéine F en diffère en outre par :
- remplacement de l'acide aminé qui, dans la séquence de ladite protéine F, est en position 49, par l'acide aminé A, ou par
- remplacement des acides aminés qui, dans la séquence de ladite protéine F, sont en positions 402, 147, 158 et 463 par les acides aminés A, V, V et V, respectivement, ou par
- remplacement des acides aminés qui, dans la séquence de ladite protéine F, sont en positions 49, 402, 147, 158 et 463, par les acides aminés A, A, V, V et V, respectivement, ou par
- remplacement de l'acide aminé qui, dans la séquence de ladite protéine F, est en position 402, par l'acide aminé A, ou par
- remplacement de l'acide aminé qui, dans la séquence de ladite protéine F, est en position 147, par l'acide aminé V, ou par
- remplacement de l'acide aminé qui, dans la séquence de ladite protéine F, est en position 158, par l'acide aminé V, ou par
- remplacement des acides aminés qui, dans la séquence de ladite protéine F, sont en positions 147 et 158, par les acides aminés V et V, respectivement, ou par
- remplacement des acides aminés qui, dans la séquence de ladite protéine F, sont en positions 147 et 463, par les acides aminés V et V, respectivement, ou par
- remplacement des acides aminés qui, dans la séquence de ladite protéine F, sont en positions 158 et 463, par les acides aminés V et V, respectivement, ou par
- remplacement des acides aminés qui, dans la séquence de ladite protéine F, sont en positions 147, 158 et 463, par les acides aminés V, V et V, respectivement.

9. La protéine mutante selon l'une quelconque des revendications 2 à 4 et 7 à 8, **caractérisée en ce que** la séquence de ladite protéine F est la séquence de SEQ ID NO : 31 avec l'acide aminé S en position 443, et **en ce que** ladite séquence qui diffère de celle de ladite protéine F en diffère en outre par :
- remplacement de l'acide aminé qui, dans la séquence de ladite protéine F, est en position 49, par l'acide aminé A, ou par
- remplacement des acides aminés qui, dans la séquence de ladite protéine F, sont en positions 402, 147, 158 et 463 par les acides aminés A, V, V et V, respectivement, ou par
- remplacement des acides aminés qui, dans la séquence de ladite protéine F, sont en positions 49, 402, 147, 158 et 463, par les acides aminés A, A, V, V et V, respectivement, ou par
- remplacement de l'acide aminé qui, dans la séquence de ladite protéine F, est en position 402, par l'acide aminé A, ou par
- remplacement de l'acide aminé qui, dans la séquence de ladite protéine F, est en position 147, par l'acide aminé V, ou par
- remplacement de l'acide aminé qui, dans la séquence de ladite protéine F, est en position 158, par l'acide aminé V, ou par
- remplacement des acides aminés qui, dans la séquence de ladite protéine F, sont en positions 147 et 158, par les acides aminés V et V, respectivement, ou par
- remplacement des acides aminés qui, dans la séquence de ladite protéine F, sont en positions 147 et 463, par les acides aminés V et V, respectivement, ou par
- remplacement des acides aminés qui, dans la séquence de ladite protéine F, sont en positions 158 et 463, par les acides aminés V et V, respectivement, ou par
- remplacement des acides aminés qui, dans la séquence de ladite protéine F, sont en positions 147, 158 et 463, par les acides aminés V, V et V, respectivement, ou par
- remplacement des acides aminés qui, dans la séquence de ladite protéine F, sont en positions 49 et 158, par les acides aminés A et V, respectivement, ou par
- remplacement des acides aminés qui, dans la séquence de ladite protéine F, sont en positions 49, 147 et 158, par les acides aminés A, V et V, respectivement, ou par
- remplacement des acides aminés qui, dans la séquence de ladite protéine F, sont en positions 49, 147, 158 et 463, par les acides aminés A, V, V et V, respectivement.

10. La protéine mutante selon l'une quelconque des revendications 2 à 5 et 7 à 9, **caractérisée en ce que** ladite séquence qui diffère de celle de ladite protéine F en diffère en outre par :
- le remplacement de l'acide aminé en position 147 par un acide aminé hydrophobe, et
- le remplacement de l'acide aminé en position 158 par un acide aminé hydrophobe.

11. La protéine mutante selon la revendication 10, **caractérisée en ce que** ladite séquence qui diffère de celle de ladite protéine F est la séquence de SEQ ID NO: 78.

12. La protéine mutante selon l'une quelconque des revendications 2 à 4 et 7 à 10, **caractérisée en ce que** ladite séquence qui diffère de celle de ladite protéine F en diffère en outre par :
- au moins une mutation de post-fusion choisie parmi :
- le remplacement de l'acide aminé en position 147 par un acide aminé hydrophobe,
- le remplacement de l'acide aminé en position 158 par un acide aminé hydrophobe ;
et
- au moins une autre mutation de post-fusion, à savoir le remplacement de l'acide aminé en position 463 par un acide aminé hydrophobe, de préférence V, I ou L, plus préférentiellement V.

13. La protéine mutante selon la revendication 1, **caractérisée en ce que** la séquence de ladite protéine F est une protéine F de PIV-2.

14. La protéine mutante selon la revendication 13, **caractérisée en ce que** la séquence de ladite protéine F consiste en :
- la séquence de SEQ ID NO: 33, ou en
- une séquence variante de cette séquence de SEQ ID NO: 33, cette séquence variante :
∘ étant d'une taille identique à celle de SEQ ID NO: 33, ou étant d'une taille supérieure d'un maximum de 2 acides aminés à celle de SEQ ID NO: 33, ou étant d'une taille inférieure d'un maximum de 2 acides aminés à celle de SEQ ID NO: 33, et
∘ présentant une identité de séquence de plus de 95%, préférentiellement d'au moins 96%, plus préférentiellement d'au moins 97%, par rapport à la séquence de SEQ ID NO: 33, cette identité étant calculée sur la longueur de la séquence de SEQ ID NO: 33.

15. La protéine mutante selon la revendication 13 ou 14, **caractérisée en ce que** ladite séquence qui diffère de celle de ladite protéine F en diffère en outre par :
- remplacement de l'acide aminé qui, dans la séquence de ladite protéine F, est en position 53, par l'acide aminé A, ou par
- remplacement des acides aminés qui, dans la séquence de ladite protéine F, sont en positions 406, 151, 162 et 474, par les acides aminés A, V, V et V, respectivement, ou par
- remplacement des acides aminés qui, dans la séquence de ladite protéine F, sont en positions 53, 406, 151, 162 et 474, par les acides aminés A, A, V, V et V, respectivement, ou par
- remplacement de l'acide aminé qui, dans la séquence de ladite protéine F, est en position 406, par l'acide aminé A, ou par
- remplacement de l'acide aminé qui, dans la séquence de ladite protéine F, est en position 151, par l'acide aminé V, respectivement, ou par
- remplacement de l'acide aminé qui, dans la séquence de ladite protéine F, est en position 162, par l'acide aminé V, respectivement, ou par
- remplacement des acides aminés qui, dans la séquence de ladite protéine F, sont en positions 151 et 162, par les acides aminés V et V, respectivement, ou par
- remplacement des acides aminés qui, dans la séquence de ladite protéine F, sont en positions 151 et 474, par les acides aminés V et V, respectivement, ou par
- remplacement des acides aminés qui, dans la séquence de ladite protéine F, sont en positions 162 et 474, par les acides aminés V et V, respectivement, ou par
- remplacement des acides aminés qui, dans la séquence de ladite protéine F, sont en positions 151, 162 et 474, par les acides aminés V, V et V, respectivement.

16. La protéine mutante selon l'une quelconque des revendications 13 à 15, **caractérisée en ce que** sa séquence en acides aminés ne comprend pas d'autre mutation par rapport à ladite séquence de protéine F de PIV-2.

17. La protéine mutante selon l'une quelconque des revendications 13 à 15, **caractérisée en ce que** ladite séquence qui diffère de celle de ladite protéine F en diffère en outre par :
- au moins une mutation de pré-fusion choisie parmi :
- le remplacement de l'acide aminé en position 53 par l'acide aminé A,
- le remplacement de l'acide aminé en position 406 par l'acide aminé A,
- le remplacement de l'acide aminé en position 428 par l'acide aminé P,
- le remplacement de l'acide aminé en position 445 par l'acide aminé P,
et/ou par
- au moins une mutation de post-fusion choisie parmi :
- le remplacement de l'acide aminé en position 151 par un acide aminé hydrophobe,
- le remplacement de l'acide aminé en position 162 par un acide aminé hydrophobe.

18. La protéine mutante selon l'une quelconque des revendications 13 à 15 et 17, **caractérisée en ce que** ladite séquence qui diffère de celle de ladite protéine F en diffère en outre par :
- au moins une mutation de post-fusion choisie parmi :
- le remplacement de l'acide aminé en position 151 par un acide aminé hydrophobe,
- le remplacement de l'acide aminé en position 162 par un acide aminé hydrophobe ;
et
- au moins une autre mutation de post-fusion, à savoir le remplacement de l'acide aminé en position 474 par un acide aminé hydrophobe, de préférence V, I ou L, plus préférentiellement V.

19. La protéine mutante selon l'une quelconque des revendications 7, 10, 12, 17, 18, **caractérisée en ce que** ledit acide aminé hydrophobe est choisi parmi V, I, L, préférentiellement V.

20. La protéine mutante selon l'une quelconque des revendications 1 à 19, **caractérisée en ce qu'**elle comprend une séquence qui diffère de celle de la protéine F d'un virus PIV-5 ou PIV-2 par substitution du site de clivage natif de ladite protéine F par un autre site de clivage enzymatique, et/ou par insertion dans ladite protéine F d'un site de clivage enzymatique autre que le site de clivage natif de cette protéine F.

21. La protéine mutante selon la revendication 20, **caractérisée en ce que** ledit site de clivage autre que le site de clivage natif est un site de clivage tissu-spécifique.

22. La protéine mutante selon l'une quelconque des revendications 1 à 21, **caractérisée en ce qu'**elle est fusogène.

23. Acide nucléique, ADN ou ARN, qui code une protéine mutante selon l'une quelconque des revendications 1 à 22, ou acide nucléique complémentaire d'un tel acide nucléique.

24. Vecteur de transfection, transduction ou transformation, comprenant au moins un acide nucléique selon la revendication 23.

25. Le vecteur d'expression selon la revendication 24, **caractérisé en ce qu'**il est un vecteur adénoviral oncolytique.

26. Cellule, **caractérisée en ce qu'**elle comprend au moins une protéine mutante selon l'une quelconque des revendications 1 à 22, et/ou au moins un acide nucléique, ADN ou ARN selon la revendication 23, et/ou au moins un vecteur selon la revendication 24 ou 25.

27. Une protéine mutante selon l'une quelconque des revendications 1 à 22, un acide nucléique, ADN ou ARN selon la revendication 23, un vecteur selon la revendication 24 ou 25, une cellule selon la revendication 26, pour une utilisation dans le traitement et/ou la prévention et/ou la palliation d'une maladie ou d'un état néoplasique, préférentiellement d'une tumeur métastasique, préférentiellement d'un mélanome métastasique.

28. Une protéine mutante selon l'une quelconque des revendications 1 à 22, un acide nucléique, ADN ou ARN selon la revendication 23, un vecteur selon la revendication 24, une cellule selon la revendication 26, pour une utilisation dans le traitement et/ou la prévention et/ou la palliation d'une déficience du développement placentaire.

29. Inhibiteur de la fusion cellulaire, **caractérisé en ce qu'**il est :
- un anticorps dirigé contre une protéine mutante selon l'une quelconque des revendications 1 à 22, ou un fragment Fab ou F(ab')2 d'un tel anticorps,
- un acide nucléique aptamère ou un peptide aptamère qui se lie spécifiquement à au moins une protéine mutante selon l'une quelconque des revendications 1 à 22, ou à un acide nucléique, ADN, ARN selon la revendication 23,
- une cellule du système immunitaire recombinante, préférentiellement une cellule dendritique recombinante, qui exprime à sa surface au moins une protéine mutante selon l'une quelconque des revendications 1 à 22,
- un acide nucléique anti-sens d'un acide nucléique selon la revendication 23,
- un petit ARN interférant (*small interfering RNA ; siRNA)* comprenant un ARN double-brin de 19 à 22 nucléotides, capable de se lier à un acide nucléique selon la revendication 23.

30. La cellule de la revendication 26, qui est une cellule du système immunitaire humain ou animal non-humain, préférentiellement une cellule dendritique humaine ou animale non-humaine, ladite cellule exprimant à sa surface au moins une protéine mutante selon l'une quelconque des revendications 1 à 22.

31. L'inhibiteur de fusion cellulaire de la revendication 29, pour une utilisation dans le traitement et/ou la prévention et/ou la palliation d'une maladie ou d'un état impliquant un excès de fusogénicité cellulaire, ladite maladie ou état étant une infection à virus enveloppé (préférentiellement une infection à VIH et/ou *Influenza* et/ou *Parainfluenza* et/ou *Rhabdovirus*), une allergie, une maladie auto-immune, un rejet de greffe.

32. Une protéine mutante selon l'une quelconque des revendications 1 à 22, pour une utilisation à titre d'agent immunogène dans le traitement et/ou la prévention et/ou la palliation d'une maladie ou d'un état impliquant un excès de fusogénicité cellulaire, ladite maladie ou d'un état étant une infection à virus enveloppé (préférentiellement une infection à VIH et/ou *Influenza* et/ou *Parainfluenza* et/ou *Rhabdovirus*), une allergie, une maladie auto-immune, un rejet de greffe.

33. Vaccin destiné au traitement et/ou à la prévention et/ou à la palliation d'une maladie ou d'un état impliquant un excès de fusogénicité cellulaire, ladite maladie ou état étant une infection à virus enveloppé (préférentiellement une infection à VIH et/ou *Influenza* et/ou *Parainfluenza* et/ou *Rhabdovirus*), une allergie, une maladie auto-immune, un rejet de greffe, **caractérisé en ce qu'**il comprend au moins protéine mutante selon l'une quelconque des revendications 1 à 22.

34. Méthode *in vitro* pour le diagnostic ou le pronostic d'une maladie impliquant une formation insuffisante, ou au contraire excessive, de syncytia, **caractérisée en ce qu'**elle comprend la détection dans un échantillon biologique d'au moins une protéine mutante selon l'une quelconque des revendications 1 à 22 ou d'au moins un acide nucléique selon la revendication 23.

35. Méthode *in vitro* pour le criblage d'un composé capable de diminuer ou bloquer la formation de syncytia, **caractérisée en ce qu'**elle comprend la mise en contact d'un composé candidat avec des cellules exprimant au moins une protéine mutante selon l'une quelconque des revendications 1 à 22, de sorte à déterminer si ce composé candidat diminue ou bloque la fusion desdites cellules.

36. Cellule tumorale, plus particulièrement myélome, comprenant au moins une protéine mutante selon l'une quelconque des revendications 1 à 22, préférentiellement comprenant au moins une telle protéine mutante à sa surface, et/ou comprenant au moins un acide nucléique selon la revendication 23, et/ou comprenant au moins un vecteur selon la revendication 24.

37. La cellule tumorale, plus particulièrement le myélome, selon la revendication 36, pour une utilisation dans la production d'un hybridome.

38. Hybridome, **caractérisé en ce qu'**il comprend au moins une protéine mutante selon l'une quelconque des revendications 1 à 22, et/ou au moins un acide nucléique selon la revendication 23, et/ou au moins un vecteur selon la revendication 24.

39. Cellule souche ou progénitrice, présentant une capacité de différentiation en cellule musculaire, **caractérisée en ce qu'**elle comprend au moins une protéine mutante selon l'une quelconque des revendications 1 à 22, préférentiellement comprenant au moins une telle protéine mutante à sa surface, et/ou comprenant au moins un acide nucléique selon la revendication 23, et/ou comprenant au moins un vecteur selon la revendication 24, ladite cellule n'étant pas une cellule embryonnaire humaine.

40. La cellule souche ou progénitrice de la revendication 39, pour une utilisation en tant que cellule souche ou progénitrice de fibre musculaire.

## Patentansprüche

1. Mutiertes Protein, dessen Aminosäuresequenz eine Sequenz umfasst, die von der des F-Proteins eines PIV-5- oder PIV-2-Virus abweicht:
- durch Austausch:
- der Aminosäure, die sich in der Sequenz des F-Proteins des PIV-5-Virus in Position 22 befindet oder die sich in der Sequenz des F-Proteins des PIV-2-Virus in Position 24 befindet, gegen die Aminosäure P, und
- der Aminosäure, die sich in der Sequenz des F-Proteins des PIV-5-Virus in Position 132 befindet oder die sich in der Sequenz des F-Proteins des PIV-2-Virus in Position 133 befindet, gegen die Aminosäure E, und
- der Aminosäure, die sich in der Sequenz des F-Proteins des PIV-5-Virus in Position 290 befindet oder die sich in der Sequenz des F-Proteins des PIV-2-Virus in Position 294 befindet, gegen die Aminosäure A, und
- der Aminosäure, die sich in der Sequenz des F-Proteins des PIV-5-Virus in Position 449 befindet oder die sich in der Sequenz des F-Proteins des PIV-2-Virus in Position 439 befindet, gegen die Aminosäure P,
- und optional:
- durch Substitution der nativen Spaltungsstelle des F-Proteins durch eine andere enzymatische Spaltungsstelle, und/oder durch Insertion in das F-Protein einer anderen enzymatischen Spaltungsstelle als der nativen Spaltungsstelle des F-Proteins, und/oder
- durch Deletion eines C-terminalen Teils des F-Proteins, wobei der C-terminale Teil sich in N-terminaler Richtung erstreckt ausgehend von der letzten Aminosäure am C-Terminus des Proteins, ohne sich dabei jedoch über die HR2-Domäne des F-Proteins hinaus zu erstrecken,
wobei die Sequenz des F-Proteins des PIV-5- oder PIV-2-Virus die Sequenz SEQ ID NO: 34 umfasst.

2. Mutiertes Protein nach Anspruch 1,
**dadurch gekennzeichnet, dass** die Sequenz des F-Proteins ein F-Protein des PIV-5 ist.

3. Mutiertes Protein nach Anspruch 2,
**dadurch gekennzeichnet, dass** die Sequenz des F-Proteins
- aus der Sequenz SEQ ID NO: 31 oder der alternativen Sequenz besteht, die mit der Sequenz SEQ ID NO: 31 mit Ausnahme der Aminosäure in Position 443, bei der es sich in dieser alternativen Sequenz um S (statt P in SEQ ID NO: 31) handelt, identisch ist, oder
- aus einer Sequenzvariante dieser Sequenz SEQ ID NO: 31 oder der alternativen Sequenz besteht, wobei diese Sequenzvariante
° ebenso groß ist wie SEQ ID NO: 31 oder um maximal 7 Aminosäuren größer ist als SEQ ID NO: 31 oder um maximal 7 Aminosäuren kleiner ist als SEQ ID NO: 31, und
° eine Sequenzidentität von mehr als 95 %, vorzugsweise wenigstens 96 %, besonders bevorzugt wenigstens 97 % gegenüber der Sequenz SEQ ID NO: 31 oder der alternativen Sequenz aufweist, wobei die Identität über die Länge der Sequenz SEQ ID NO: 31 oder gegebenenfalls der alternativen Sequenz gerechnet ist.

4. Mutiertes Protein nach Anspruch 3,
**dadurch gekennzeichnet, dass** die Sequenz des F-Proteins aus der Sequenz SEQ ID NO: 31 besteht, modifiziert durch die in Tabelle 1 in Spalte "W3A" oder "MIL" oder "DEN" oder "LN" oder "MEL" oder "Cryptovirus" oder "CPI+" oder "CPI-" oder "H221" oder "78524" oder "T1" oder "SER" angegebenen Aminosäureaustausche.

5. Mutiertes Protein nach einem der Ansprüche 2 bis 4,
**dadurch gekennzeichnet, dass** die Aminosäuresequenz keine weitere Mutation bezüglich der Sequenz des F-Proteins des PIV-5 umfasst.

6. Mutiertes Protein nach Anspruch 5,
**dadurch gekennzeichnet, dass** diese Sequenz, die von der des F-Proteins abweicht, die Sequenz SEQ ID NO: 65 ist.

7. Mutiertes Protein nach einem der Ansprüche 2 bis 4,
**dadurch gekennzeichnet, dass** diese Sequenz, die von der des F-Proteins abweicht, sich zudem von dieser unterscheidet durch:
- wenigstens eine Präfusionsmutation gewählt aus:
- dem Austausch der Aminosäure in Position 49 gegen die Aminosäure A,
- dem Austausch der Aminosäure in Position 402 gegen die Aminosäure A,
- dem Austausch der Aminosäure in Position 443 gegen die Aminosäure P,
- dem Austausch der Aminosäure in Position 447 gegen die Aminosäure P, und/oder durch
- wenigstens eine Postfusionsmutation gewählt aus:
- dem Austausch der Aminosäure in Position 147 gegen eine hydrophobe Aminosäure,
- dem Austausch der Aminosäure in Position 158 gegen eine hydrophobe Aminosäure.

8. Mutiertes Protein nach einem der Ansprüche 2 bis 4 und 7,
**dadurch gekennzeichnet, dass** diese Sequenz, die von der des F-Proteins abweicht, sich zudem von dieser unterscheidet durch:
- Austausch der Aminosäure, die sich in der Sequenz des F-Proteins in Position 49 befindet, gegen die Aminosäure A, oder durch
- Austausch der Aminosäuren, die sich in der Sequenz des F-Proteins in Position 402, 147, 158 und 463 befinden, gegen die Aminosäuren A, V, V bzw. V oder durch
- Austausch der Aminosäuren, die sich in der Sequenz des F-Proteins in Position 49, 402, 147, 158 und 463 befinden, gegen die Aminosäuren A, A, V, V bzw. V oder durch
- Austausch der Aminosäure, die sich in der Sequenz des F-Proteins in Position 402 befindet, gegen die Aminosäure A, oder durch
- Austausch der Aminosäure, die sich in der Sequenz des F-Proteins in Position 147 befindet, gegen die Aminosäure V, oder durch
- Austausch der Aminosäure, die sich in der Sequenz des F-Proteins in Position 158 befindet, gegen die Aminosäure V, oder durch
- Austausch der Aminosäuren, die sich in der Sequenz des F-Proteins in Position 147 und 158 befinden, gegen die Aminosäuren V bzw. V oder durch
- Austausch der Aminosäuren, die sich in der Sequenz des F-Proteins in Position 147 und 463 befinden, gegen die Aminosäuren V bzw. V oder durch
- Austausch der Aminosäuren, die sich in der Sequenz des F-Proteins in Position 158 und 463 befinden, gegen die Aminosäuren V bzw. V oder durch
- Austausch der Aminosäuren, die sich in der Sequenz des F-Proteins in Position 147, 158 und 463 befinden, gegen die Aminosäuren V, V bzw. V.

9. Mutiertes Protein nach einem der Ansprüche 2 bis 4 und 7 bis 8,
**dadurch gekennzeichnet, dass** die Sequenz des F-Proteins die Sequenz SEQ ID NO: 31 ist mit der Aminosäure S in Position 443, und dass diese Sequenz, die von der des F-Proteins abweicht, sich zudem von dieser unterscheidet durch:
- Austausch der Aminosäure, die sich in der Sequenz des F-Proteins in Position 49 befindet, gegen die Aminosäure A, oder durch
- Austausch der Aminosäuren, die sich in der Sequenz des F-Proteins in Position 402, 147, 158 und 463 befinden, gegen die Aminosäuren A, V, V bzw. V oder durch
- Austausch der Aminosäuren, die sich in der Sequenz des F-Proteins in Position 49, 402, 147, 158 und 463 befinden, gegen die Aminosäuren A, A, V, V bzw. V oder durch
- Austausch der Aminosäure, die sich in der Sequenz des F-Proteins in Position 402 befindet, gegen die Aminosäure A, oder durch
- Austausch der Aminosäure, die sich in der Sequenz des F-Proteins in Position 147 befindet, gegen die Aminosäure V, oder durch
- Austausch der Aminosäure, die sich in der Sequenz des F-Proteins in Position 158 befindet, gegen die Aminosäure V, oder durch
- Austausch der Aminosäuren, die sich in der Sequenz des F-Proteins in Position 147 und 158 befinden, gegen die Aminosäuren V bzw. V oder durch
- Austausch der Aminosäuren, die sich in der Sequenz des F-Proteins in Position 147 und 463 befinden, gegen die Aminosäuren V bzw. V oder durch
- Austausch der Aminosäuren, die sich in der Sequenz des F-Proteins in Position 158 und 463 befinden, gegen die Aminosäuren V bzw. V oder durch
- Austausch der Aminosäuren, die sich in der Sequenz des F-Proteins in Position 147, 158 und 463 befinden gegen die Aminosäuren V, V bzw. V oder durch
- Austausch der Aminosäuren, die sich in der Sequenz des F-Proteins in Position 49 und 158 befinden, gegen die Aminosäuren A bzw. V oder durch
- Austausch der Aminosäuren, die sich in der Sequenz des F-Proteins in Position 49, 147 und 158 befinden, gegen die Aminosäuren A, V bzw. V oder durch
- Austausch der Aminosäuren, die sich in der Sequenz des F-Proteins in Position 49, 147, 158 und 463 befinden, gegen die Aminosäuren A, V, V bzw. V.

10. Mutiertes Protein nach einem der Ansprüche 2 bis 4 und 7 bis 9,
**dadurch gekennzeichnet, dass** diese Sequenz, die von der des F-Proteins abweicht, sich zudem von dieser unterscheidet durch:
- den Austausch der Aminosäure in Position 147 gegen eine hydrophobe Aminosäure, und
- den Austausch der Aminosäure in Position 158 gegen eine hydrophobe Aminosäure.

11. Mutiertes Protein nach Anspruch 10,
**dadurch gekennzeichnet, dass** diese Sequenz, die von der des F-Proteins abweicht, die Sequenz SEQ ID NO: 78 ist.

12. Mutiertes Protein nach einem der Ansprüche 2 bis 4 und 7 bis 10,
**dadurch gekennzeichnet, dass** diese Sequenz, die von der des F-Proteins abweicht, sich zudem von dieser unterscheidet durch:
- wenigstens eine Postfusionsmutation gewählt aus:
- dem Austausch der Aminosäure in Position 147 gegen eine hydrophobe Aminosäure,
- dem Austausch der Aminosäure in Position 158 gegen eine hydrophobe Aminosäure;
und
- wenigstens eine andere Postfusionsmutation, nämlich den Austausch der Aminosäure in Position 463 gegen eine hydrophobe Aminosäure, vorzugsweise V, I oder L, besonders bevorzugt V.

13. Mutiertes Protein nach Anspruch 1,
**dadurch gekennzeichnet, dass** die Sequenz des F-Proteins ein F-Protein des PIV-2 ist.

14. Mutiertes Protein nach Anspruch 13,
**dadurch gekennzeichnet, dass** die Sequenz des F-Proteins
- aus der Sequenz SEQ ID NO: 33 oder
- aus einer Sequenzvariante dieser Sequenz SEQ ID NO: 33 besteht, wobei diese Sequenzvariante
∘ ebenso groß ist wie SEQ ID NO: 33 oder um maximal 2 Aminosäuren größer ist als SEQ ID NO: 33 oder um maximal 2 Aminosäuren kleiner ist als SEQ ID NO: 33, und
∘ eine Sequenzidentität von mehr als 95 %, vorzugsweise wenigstens 96 %, besonders bevorzugt wenigstens 97 % gegenüber der Sequenz SEQ ID NO: 33 aufweist, wobei die Identität über die Länge der Sequenz SEQ ID NO: 33 gerechnet ist.

15. Mutiertes Protein nach Anspruch 13 oder 14,
**dadurch gekennzeichnet, dass** diese Sequenz, die von der des F-Proteins abweicht, sich zudem von dieser unterscheidet durch:
- Austausch der Aminosäure, die sich in der Sequenz des F-Proteins in Position 53 befindet, gegen die Aminosäure A, oder durch
- Austausch der Aminosäuren, die sich in der Sequenz des F-Proteins in Position 406, 151, 162 und 474 befinden, gegen die Aminosäuren A, V, V bzw. V oder durch
- Austausch der Aminosäuren, die sich in der Sequenz des F-Proteins in Position 53, 406, 151, 162 und 474 befinden, gegen die Aminosäuren A, A, V, V bzw. V oder durch
- Austausch der Aminosäure, die sich in der Sequenz des F-Proteins in Position 406 befindet, gegen die Aminosäure A, oder durch
- Austausch der Aminosäure, die sich in der Sequenz des F-Proteins in Position 151 befindet, gegen die Aminosäure V, oder durch
- Austausch der Aminosäure, die sich in der Sequenz des F-Proteins in Position 162 befindet, gegen die Aminosäure V, oder durch
- Austausch der Aminosäuren, die sich in der Sequenz des F-Proteins in Position 151 und 162 befinden, gegen die Aminosäuren V bzw. V oder durch
- Austausch der Aminosäuren, die sich in der Sequenz des F-Proteins in Position 151 und 474 befinden, gegen die Aminosäuren V bzw. V oder durch
- Austausch der Aminosäuren, die sich in der Sequenz des F-Proteins in Position 162 und 474 befinden, gegen die Aminosäuren V bzw. V oder durch
- Austausch der Aminosäuren, die sich in der Sequenz des F-Proteins in Position 151, 162 und 474 befinden, gegen die Aminosäuren V, V bzw. V.

16. Mutiertes Protein nach einem der Ansprüche 13 bis 15,
**dadurch gekennzeichnet, dass** seine Aminosäuresequenz keine weitere Mutation bezüglich der Sequenz des F-Proteins des PIV-2 umfasst.

17. Mutiertes Protein nach einem der Ansprüche 13 bis 15,
**dadurch gekennzeichnet, dass** diese Sequenz, die von der des F-Proteins abweicht, sich zudem von dieser unterscheidet durch:
- wenigstens eine Präfusionsmutation gewählt aus:
- dem Austausch der Aminosäure in Position 53 gegen die Aminosäure A,
- dem Austausch der Aminosäure in Position 406 gegen die Aminosäure A,
- dem Austausch der Aminosäure in Position 428 gegen die Aminosäure P,
- dem Austausch der Aminosäure in Position 445 gegen die Aminosäure P,
und/oder durch
- wenigstens eine Postfusionsmutation gewählt aus:
- dem Austausch der Aminosäure in Position 151 gegen eine hydrophobe Aminosäure,
- dem Austausch der Aminosäure in Position 162 gegen eine hydrophobe Aminosäure.

18. Mutiertes Protein nach einem der Ansprüche 13 bis 15 und 17,
**dadurch gekennzeichnet, dass** diese Sequenz, die von der des F-Proteins abweicht, sich zudem von dieser unterscheidet durch:
- wenigstens eine Postfusionsmutation gewählt aus:
- dem Austausch der Aminosäure in Position 151 gegen eine hydrophobe Aminosäure,
- dem Austausch der Aminosäure in Position 162 gegen eine hydrophobe Aminosäure;
und
- wenigstens eine andere Postfusionsmutation, nämlich den Austausch der Aminosäure in Position 474 gegen eine hydrophobe Aminosäure, vorzugsweise V, I oder L, besonders bevorzugt V.

19. Mutiertes Protein nach einem der Ansprüche 7, 10, 12, 17, 18,
**dadurch gekennzeichnet, dass** diese hydrophobe Aminosäure gewählt ist aus V, I, L, bevorzugt V.

20. Mutiertes Protein nach einem der Ansprüche 1 bis 19,
**dadurch gekennzeichnet, dass** es eine Sequenz umfasst, die von der des F-Proteins eines PIV-5- oder PIV-2-Virus abweicht durch Substitution der nativen Spaltungsstelle des F-Proteins durch eine andere enzymatische Spaltungsstelle, und/oder durch Insertion in das F-Protein einer anderen enzymatischen Spaltungsstelle als der nativen Spaltungsstelle des F-Proteins.

21. Mutiertes Protein nach Anspruch 20,
**dadurch gekennzeichnet, dass** die andere Spaltungsstelle als die native Spaltungsstelle eine gewebespezifische Spaltungsstelle ist.

22. Mutiertes Protein nach einem der Ansprüche 1 bis 21,
**dadurch gekennzeichnet, dass** es fusogen ist.

23. Nucleinsäure, DNA oder RNA, die ein mutiertes Protein nach einem der Ansprüche 1 bis 22 codiert, oder eine derartige Nucleinsäure ergänzende Nucleinsäure.

24. Transfektions-, Transduktions oder Transformationsvektor, wenigstens eine Nucleinsäure nach Anspruch 23 umfassend.

25. Expressionsvektor nach Anspruch 24,
**dadurch gekennzeichnet, dass** er ein onkolytischer adenoviraler Vektor ist.

26. Zelle,
**dadurch gekennzeichnet, dass** sie wenigstens ein mutiertes Protein nach einem der Ansprüche 1 bis 22 und/oder wenigstens eine Nucleinsäure, DNA oder RNA, nach Anspruch 23 und/oder wenigstens einen Vektor nach Anspruch 24 oder 25 umfasst.

27. Mutiertes Protein nach einem der Ansprüche 1 bis 22, Nucleinsäure, DNA oder RNA, nach Anspruch 23, Vektor nach Anspruch 24 oder 25, Zelle nach Anspruch 26, zur Verwendung bei der Behandlung und/oder Prävention und/oder Palliation einer neoplastischen Erkrankung oder eines neoplastischen Zustands, bevorzugt eines metastasierenden Tumors, bevorzugt eines metastasierenden Melanoms.

28. Mutiertes Protein nach einem der Ansprüche 1 bis 22, Nucleinsäure, DNA oder RNA, nach Anspruch 23, Vektor nach Anspruch 24, Zelle nach Anspruch 26, zur Verwendung bei der Behandlung und/oder Prävention und/oder Palliation einer mangelhaften Plazentaentwicklung.

29. Zellfusionsinhibitor,
**dadurch gekennzeichnet, dass** es sich bei diesem handelt um:
- einen gegen ein mutiertes Protein nach einem der Ansprüche 1 bis 22 gerichteten Antikörper, oder ein Fab- oder F(ab')2-Fragment eines derartigen Antikörpers,
- ein Nucleinsäure-Aptamer oder ein Peptid-Aptamer, das sich typischerweise an wenigstens ein mutiertes Protein nach einem der Ansprüche 1 bis 22 oder eine Nucleinsäure, DNA oder RNA, nach Anspruch 23 bindet,
- eine rekombinante Zelle des Immunsystems, vorzugsweise eine rekombinante dendritische Zelle, die an ihrer Oberfläche wenigstens ein mutiertes Protein nach einem der Ansprüche 1 bis 22 exprimiert,
- eine Antisense-Nucleinsäure einer Nucleinsäure nach Anspruch 23,
- eine *small interfering* RNA (*siRNA*) mit einer Doppelstrang-RNA mit 19 bis 22 Nucleotiden, die sich an eine Nucleinsäure nach Anspruch 23 zu binden vermag.

30. Zelle nach Anspruch 26, bei der es sich um eine Zelle des humanen oder nicht humanen tierischen Immunsystems, vorzugsweise eine humane oder nicht humane tierische dendritische Zelle handelt, wobei die Zelle an ihrer Oberfläche wenigstens ein mutiertes Protein nach einem der Ansprüche 1 bis 22 exprimiert.

31. Zellfusionsinhibitor nach Anspruch 29, zur Verwendung bei der Behandlung und/oder Prävention und/oder Palliation einer Erkrankung oder eines Zustands, die bzw. der ein Übermaß an Zellfusogenität impliziert, wobei es sich bei der Erkrankung oder dem Zustand um eine Infektion mit einem umhüllten Virus (bevorzugt eine HIV-und/oder Influenza- und/oder Parainfluenza- und/oder Rhabdovirus-Infektion), eine Allergie, eine Autoimmunerkrankung, eine Transplantatabstoßung handelt.

32. Mutiertes Protein nach einem der Ansprüche 1 bis 22, zur Verwendung als Immunogen bei der Behandlung und/oder Prävention und/oder Palliation einer Erkrankung oder eines Zustands, die bzw. der ein Übermaß an Zellfusogenität impliziert, wobei es sich bei der Erkrankung oder dem Zustand um eine Infektion mit einem umhüllten Virus (bevorzugt eine HIV- und/oder Influenza- und/oder Parainfluenza- und/oder Rhabdovirus-Infektion), eine Allergie, eine Autoimmunerkrankung, eine Transplantatabstoßung handelt.

33. Impfstoff zur Behandlung und/oder Prävention und/oder Palliation einer Erkrankung oder eines Zustands, die bzw. der ein Übermaß an Zellfusogenität impliziert wobei es sich bei der Erkrankung oder dem Zustand um eine Infektion mit einem umhüllten Virus (bevorzugt eine HIV- und/oder Influenza- und/oder Parainfluenza- und/oder Rhabdovirus-Infektion), eine Allergie, eine Autoimmunerkrankung, eine Transplantatabstoßung handelt,
**dadurch gekennzeichnet, dass** er wenigstens ein mutiertes Protein nach einem der Ansprüche 1 bis 22 umfasst.

34. In-vitro-Verfahren zur Diagnose oder Prognose einer Erkrankung, die eine ungenügende oder im Gegenteil eine übermäßige Bildung von Synzytien impliziert, **dadurch gekennzeichnet, dass** es den Nachweis in einer biologischen Probe von wenigstens einem mutierten Protein nach einem der Ansprüche 1 bis 22 oder wenigstens einer Nucleinsäure nach Anspruch 23 umfasst.

35. In-vitro-Verfahren zum Screenen einer Verbindung, die die Bildung von Synzytien zu reduzieren oder blockieren vermag,
**dadurch gekennzeichnet, dass** es das Inkontaktbringen einer Kandidatenverbindung mit Zellen umfasst, die wenigstens ein mutiertes Protein nach einem der Ansprüche 1 bis 22 exprimieren, so dass bestimmt werden kann, ob diese Kandidatenverbindung die Fusion dieser Zellen reduziert oder blockiert.

36. Tumorzelle, insbesondere Myelomzelle, wenigstens ein mutiertes Protein nach einem der Ansprüche 1 bis 22 umfassend, vorzugsweise wenigstens ein derartiges mutiertes Protein an ihrer Oberfläche umfassend und/oder wenigstens eine Nucleinsäure nach Anspruch 23 umfassend und/oder wenigstens einen Vektor nach Anspruch 24 umfassend.

37. Tumorzelle, insbesondere Myelomzelle, nach Anspruch 36, zur Verwendung bei der Herstellung eines Hybridoms.

38. Hybridom,
**dadurch gekennzeichnet, dass** es wenigstens ein mutiertes Protein nach einem der Ansprüche 1 bis 22 und/oder wenigstens eine Nucleinsäure nach Anspruch 23 und/oder wenigstens einen Vektor nach Anspruch 24 umfasst.

39. Stammzelle oder Vorläuferzelle, die eine Differenzierungsfähigkeit in Muskelzellen aufweist,
**dadurch gekennzeichnet, dass** sie wenigstens ein mutiertes Protein nach einem der Ansprüche 1 bis 22 umfasst, vorzugsweise wenigstens ein derartiges mutiertes Protein an ihrer Oberfläche umfassend und/oder wenigstens eine Nucleinsäure nach Anspruch 23 umfassend und/oder wenigstens einen Vektor nach Anspruch 24 umfassend, wobei die Zelle keine humane embryonale Zelle ist.

40. Stammzelle oder Vorläuferzelle nach Anspruch 39, zur Verwendung als Muskelfaserstammzelle oder -vorläuferzelle.

## Claims

1. A mutant protein the amino acid sequence of which comprises a sequence which differs from that of the F protein of a PIV-5 or PIV-2 virus:
• by replacement:
∘ of the amino acid which in the sequence for said F protein of the PIV-5 virus, is in position 22, or which, in the sequence for said F protein of the PIV-2 virus, is in position 24, by the amino acid P; and
∘ of the amino acid which in the sequence for said F protein of the PIV-5 virus, is in position 132, or which, in the sequence for said F protein of the PIV-2 virus, is in position 133, by the amino acid E; and
∘ of the amino acid which in the sequence for said F protein of the PIV-5 virus, is in position 290, or which, in the sequence for said F protein of the PIV-2 virus, is in position 294, by the amino acid A; and
∘ of the amino acid which in the sequence for said F protein of the PIV-5 virus, is in position 449, or which, in the sequence for said F protein of the PIV-2 virus, is in position 439, by the amino acid P;
• and optionally:
o by substitution of the native cleavage site of said F protein by another enzymatic cleavage site, and/or by insertion into said F protein of an enzymatic cleavage site other than the native cleavage site of said F protein; and/or
o by deletion of a C-terminal portion of said F protein, said C-terminal portion extending in the N-terminal direction from the last amino acid at the C-terminal end of the protein, but without extending beyond the HR2 domain of said F protein;
the sequence for said F protein of the PIV-5 or PIV-2 virus comprising the sequence SEQ ID NO: 34.

2. The mutant protein as claimed in claim 1, **characterized in that** the sequence for said F protein is a F protein of PIV-5.

3. The mutant protein as claimed in claim 2, **characterized in that** the sequence for said F protein consists of:
• the sequence SEQ ID NO: 31, or the alternative sequence which is identical to the sequence SEQ ID NO: 31 with the exception of the amino acid in position 443, which is S in this alternative sequence (in place of P in SEQ ID NO: 31); or of
• a variant sequence for this sequence SEQ ID NO: 31 or of said alternative sequence, this variant sequence:
o being identical in size to that of SEQ ID NO: 31, or being of a size larger by a maximum of 7 amino acids than that of SEQ ID NO: 31, or being of a size smaller by a maximum of 7 amino acids than that of SEQ ID NO: 31; and
o having a sequence identity of more than 95%, preferably at least 96%, more preferably at least 97%, with respect to the sequence SEQ ID NO: 31 or to said alternative sequence, this identity being calculated using the length of the sequence SEQ ID NO: 31 or, if appropriate, of said alternative sequence.

4. The mutant protein as claimed in claim 3, **characterized in that** the sequence for said F protein consists of the sequence SEQ ID NO: 31 modified by replacements of the amino acids indicated in Table 1 in the column "W3A" or "MIL" or "DEN" or "LN" or "MEL" or "Cryptovirus" or "CPI+" or "CPI-" or "H221" or "78524" or "T1" or "SER".

5. The mutant protein as claimed in any one of claims 2 to 4, **characterized in that** its amino acid sequence does not comprise any other mutation with respect to said PIV-5 F protein sequence.

6. The mutant protein as claimed in claim 5, **characterized in that** said sequence which differs from that for said F protein is the sequence SEQ ID NO: 65.

7. The mutant protein as claimed in any one of claims 2 to 4, **characterized in that** said sequence which differs from that for said F protein differs further by:
• at least one pre-fusion mutation selected from:
o replacement of the amino acid in position 49 by the amino acid A;
o replacement of the amino acid in position 402 by the amino acid A;
o replacement of the amino acid in position 443 by the amino acid P;
o replacement of the amino acid in position 447 by the amino acid P;
and/or by
• at least one post-fusion mutation selected from:
o replacement of the amino acid in position 147 by a hydrophobic amino acid;
o replacement of the amino acid in position 158 by a hydrophobic amino acid.

8. The mutant protein as claimed in any one of claims 2 to 4 and 7, **characterized in that** said sequence which differs from that for said F protein differs further by:
• replacement of the amino acid which in the sequence for said F protein is in position 49, by the amino acid A; or by
• replacement of the amino acids which in the sequence for said F protein are in positions 402, 147, 158 and 463, by the amino acids A, V, V and V respectively; or by
• replacement of the amino acids which in the sequence for said F protein are in positions 49, 402, 147, 158 and 463, by the amino acids A, A, V, V and V respectively; or by
• replacement of the amino acid which in the sequence for said F protein is in position 402, by the amino acid A; or by
• replacement of the amino acid which in the sequence for said F protein is in position 147, by the amino acid V; or by
• replacement of the amino acid which in the sequence for said F protein is in position 158, by the amino acid V; or by
• replacement of the amino acids which in the sequence for said F protein are in positions 147 and 158, by the amino acids V and V respectively; or by
• replacement of the amino acids which in the sequence for said F protein are in positions 147 and 463, by the amino acids V and V respectively; or by
• replacement of the amino acids which in the sequence for said F protein are in positions 158 and 463, by the amino acids V and V respectively; or by
• replacement of the amino acids which in the sequence for said F protein are in positions 147, 158 and 463, by the amino acids V, V and V respectively.

9. The mutant protein as claimed in any one of claims 2 to 4 and 7 to 8, **characterized in that** the sequence for said F protein is the sequence SEQ ID NO: 31 with the amino acid S in position 443, and **in that** said sequence which differs from that for said F protein differs further by:
• replacement of the amino acid which in the sequence for said F protein is in position 49, by the amino acid A; or by
• replacement of the amino acids which in the sequence for said F protein are in positions 402, 147, 158 and 463, by the amino acids A, V, V and V respectively; or by
• replacement of the amino acids which in the sequence for said F protein are in positions 49, 402, 147, 158 and 463, by the amino acids A, A, V, V and V respectively; or by
• replacement of the amino acid which in the sequence for said F protein is in position 402, by the amino acid A; or by
• replacement of the amino acid which in the sequence for said F protein is in position 147, by the amino acid V; or by
• replacement of the amino acid which in the sequence for said F protein is in position 158, by the amino acid V; or by
• replacement of the amino acids which in the sequence for said F protein are in positions 147 and 158, by the amino acids V and V respectively; or by
• replacement of the amino acids which in the sequence for said F protein are in positions 147 and 463, by the amino acids V and V respectively; or by
• replacement of the amino acids which in the sequence for said F protein are in positions 158 and 463, by the amino acids V and V respectively; or by
• replacement of the amino acids which in the sequence for said F protein are in positions 147, 158 and 463, by the amino acids V, V and V respectively; or by
• replacement of the amino acids which in the sequence for said F protein are in positions 49 and 158, by the amino acids A and V respectively; or by
• replacement of the amino acids which in the sequence for said F protein are in positions 49, 147 and 158, by the amino acids A, V and V respectively; or by
• replacement of the amino acids which in the sequence for said F protein are in positions 49, 147, 158 and 463, by the amino acids A, V, V and V respectively.

10. The mutant protein as claimed in any one of claims 2 to 5 and 7 to 9, **characterized in that** said sequence which differs from that for said F protein differs further by:
• replacement of the amino acid in position 147 by a hydrophobic amino acid; and
• replacement of the amino acid in position 158 by a hydrophobic amino acid.

11. The mutant protein as claimed in claim 10, **characterized in that** said sequence which differs from that for said F protein is the sequence SEQ ID NO: 78.

12. The mutant protein as claimed in any one of claims 2 to 4 and 7 to 10, **characterized in that** said sequence which differs from that for said F protein differs further by:
• at least one post-fusion mutation selected from:
o replacement of the amino acid in position 147 by a hydrophobic amino acid;
o replacement of the amino acid in position 158 by a hydrophobic amino acid;
and
• at least one other post-fusion mutation, namely replacement of the amino acid in position 463 by a hydrophobic amino acid, preferably V, I or L, more preferably V.

13. The mutant protein as claimed in claim 1, **characterized in that** the sequence for said F protein is a PIV-2 F protein.

14. The mutant protein as claimed in claim 13, **characterized in that** the sequence for said F protein consists of:
• the sequence SEQ ID NO: 33; or of
• a variant sequence for this sequence SEQ ID NO: 33, this variant sequence:
o being identical in size to that of SEQ ID NO: 33, or being of a size larger by a maximum of 2 amino acids than that of SEQ ID NO: 33, or being of a size smaller by a maximum of 2 amino acids than that of SEQ ID NO: 33; and
o having a sequence identity of more than 95%, preferably at least 96%, more preferably at least 97%, with respect to the sequence SEQ ID NO: 33, this identity being calculated using the length of the sequence SEQ ID NO: 33.

15. The mutant protein as claimed in claim 13 or claim 14, **characterized in that** said sequence which differs from that for said F protein differs further by:
• replacement of the amino acid which in the sequence for said F protein is in position 53, by the amino acid A; or by
• replacement of the amino acids which in the sequence for said F protein are in positions 406, 151, 162 and 474, by the amino acids A, V, V and V respectively; or by
• replacement of the amino acids which in the sequence for said F protein are in positions 53, 406, 151, 162 and 474, by the amino acids A, A, V, V and V respectively; or by
• replacement of the amino acid which in the sequence for said F protein is in position 406, by the amino acid A; or by
• replacement of the amino acid which in the sequence for said F protein is in position 151, by the amino acid V; or by
• replacement of the amino acid which in the sequence for said F protein is in position 162, by the amino acid V; or by
• replacement of the amino acids which in the sequence for said F protein are in positions 151 and 162, by the amino acids V and V respectively; or by
• replacement of the amino acids which in the sequence for said F protein are in positions 151 and 474, by the amino acids V and V respectively; or by
• replacement of the amino acids which in the sequence for said F protein are in positions 162 and 474, by the amino acids V and V respectively; or by
• replacement of the amino acids which in the sequence for said F protein are in positions 151, 162 and 474, by the amino acids V, V and V respectively.

16. The mutant protein as claimed in any one of claims 13 to 15, **characterized in that** its amino acid sequence does not comprise any other mutations with respect to said PIV-2F protein sequence.

17. The mutant protein as claimed in any one of claims 13 to 15, **characterized in that** said sequence which differs from that for said F protein differs further by:
• at least one pre-fusion mutation selected from:
o replacement of the amino acid in position 53 by the amino acid A;
o replacement of the amino acid in position 406 by the amino acid A;
o replacement of the amino acid in position 428 by the amino acid P;
o replacement of the amino acid in position 445 by the amino acid P;
and/or by
• at least one post-fusion mutation selected from:
o replacement of the amino acid in position 151 by a hydrophobic amino acid;
o replacement of the amino acid in position 162 by a hydrophobic amino acid.

18. The mutant protein as claimed in any one of claims 13 to 15 and 17, **characterized in that** said sequence which differs from that for said F protein differs further by:
• at least one post-fusion mutation selected from:
• replacement of the amino acid in position 151 by a hydrophobic amino acid;
• replacement of the amino acid in position 162 by a hydrophobic amino acid; and
• at least one other post-fusion mutation, namely replacement of the amino acid in position 474 by a hydrophobic amino acid, preferably V, I or L, more preferably V.

19. The mutant protein as claimed in any one of claims 7, 10, 12, 17, 18, **characterized in that** said hydrophobic amino acid is selected from V, I, L, preferably V.

20. The mutant protein as claimed in any one of claims 1 to 19, **characterized in that** it comprises a sequence which differs from that of the F protein of a PIV-5 or PIV-2 virus by substitution of the native cleavage site of said F protein by another enzymatic cleavage site, and/or by insertion into said F protein of an enzymatic cleavage site other than the native cleavage site of this F protein.

21. The mutant protein as claimed in claim 20, **characterized in that** said cleavage site other than the native cleavage site is a tissue-specific cleavage site.

22. The mutant protein as claimed in any one of claims 1 to 21, **characterized in that** it is fusogenic.

23. A nucleic acid, DNA or RNA, which encodes a mutant protein as claimed in any one of claims 1 to 22, or complementary nucleic acid of such a nucleic acid.

24. A transfection, transduction or transformation vector, comprising at least one nucleic acid as claimed in claim 23.

25. The expression vector as claimed in claim 24, **characterized in that** it is an oncolytic adenoviral vector.

26. A cell, **characterized in that** it comprises at least one mutant protein as claimed in any one of claims 1 to 22, and/or at least one nucleic acid, DNA or RNA, as claimed in claim 23, and/or at least one vector as claimed in claim 24 or claim 25.

27. A mutant protein as claimed in any one of claims 1 to 22, a nucleic acid, DNA or RNA, as claimed in claim 23, a vector as claimed in claim 24 or claim 25, a cell as claimed in claim 26, for use in the treatment and/or prevention and/or mitigation of a disease or a neoplasic condition, preferably a metastatic tumour, preferably a metastatic melanoma.

28. A mutant protein as claimed in any one of claims 1 to 22, a nucleic acid, DNA or RNA, as claimed in claim 23, a vector as claimed in claim 24, a cell as claimed in claim 26, for use in the treatment and/or prevention and/or mitigation of a deficiency of placental development.

29. An inhibitor of cell fusion, **characterized in that** it is:
• an antibody directed against a mutant protein as claimed in any one of claims 1 to 22, or a Fab or F(ab')2 fragment of such an antibody;
• a nucleic acid aptamer or a peptide aptamer which binds specifically to at least one mutant protein as claimed in any one of claims 1 to 22, or to a nucleic acid, DNA, RNA as claimed in claim 23;
• a recombinant immune system cell, preferably a recombinant dendritic cell, which expresses at least one mutant protein as claimed in any one of claims 1 to 22 at its surface;
• an antisense nucleic acid of a nucleic acid as claimed in claim 23;
• a small interfering RNA, siRNA, comprising a double strand RNA containing 19 to 22 nucleotides, capable of binding to a nucleic acid as claimed in claim 23.

30. The cell of claim 26, which is a cell of the human or non-human animal immune system, preferably a human or non-human animal dendritic cell, said cell expressing at least one mutant protein as claimed in any one of claims 1 to 22 at its surface.

31. The cell fusion inhibitor of claim 29, for use in the treatment and/or prevention and/or mitigation of a disease or a condition involving an excess of cellular fusogenicity, said disease or condition being an enveloped virus infection (preferably a HIV and/or influenza and/or parainfluenza and/or rhabdovirus infection), an allergy, an auto-immune disease or a graft rejection.

32. A mutant protein as claimed in any one of claims 1 to 22, for use as an immunogenic agent in the treatment and/or prevention and/or mitigation of a disease or a condition involving an excess of cellular fusogenicity, said disease or condition being an enveloped virus infection (preferably a HIV and/or influenza and/or parainfluenza and/or rhabdovirus infection), an allergy, an auto-immune disease or a graft rejection.

33. A vaccine intended for the treatment and/or prevention and/or mitigation of a disease or a condition involving an excess of cellular fusogenicity, said disease or condition being an enveloped virus infection (preferably a HIV and/or influenza and/or parainfluenza and/or rhabdovirus infection), an allergy, an auto-immune disease or a graft rejection, **characterized in that** it comprises at least mutant protein as claimed in any one of claims 1 to 22.

34. *An in vitro* method for the diagnosis or prognosis of a disease involving insufficient, or in contrast excessive formation of syncytia, **characterized in that** it comprises the detection, in a biological sample, of at least one mutant protein as claimed in any one of claims 1 to 22 or of at least one nucleic acid as claimed in claim 23.

35. *An in vitro* method for screening a compound capable of reducing or blocking the formation of syncytia, **characterized in that** it comprises bringing a candidate compound into contact with at least one mutant protein as claimed in any one of claims 1 to 22, in order to determine whether said candidate compound reduces or blocks fusion of said cells.

36. A tumour cell, more particularly a myeloma, comprising at least one mutant protein as claimed in any one of claims 1 to 22, preferably comprising at least one such mutant protein on its surface, and/or comprising at least one nucleic acid as claimed in claim 23, and/or comprising at least one vector as claimed in claim 24.

37. A tumour cell, more particularly the myeloma, as claimed in claim 36, for use in the production of a hybridoma.

38. A hybridoma, **characterized in that** it comprises at least one mutant protein as claimed in any one of claims 1 to 22, and/or at least one nucleic acid as claimed in claim 23, and/or at least one vector as claimed in claim 24.

39. A stem cell or progenitor cell, having a capacity for differentiation into muscle cell, **characterized in that** it comprises at least one mutant protein as claimed in any one of claims 1 to 22, preferably comprising at least one such mutant protein on its surface, and/or comprising at least one nucleic acid as claimed in claim 23, and/or comprising at least one vector as claimed in claim 24, said cell not being a human embryo cell.

40. The stem cell or progenitor cell of claim 39, for use as a muscle fibre stem cell or progenitor cell.
